# EUROPÄISCHE PATENTSCHRIFT

(11) **EP 1 594 986 B1**
(45) Veröffentlichungstag und Bekanntmachung des Hinweises auf die Patenterteilung: **13.07.2011**
(21) Anmeldenummer: 04712508.3
(22) Anmeldetag: 19.02.2004
(51) Int. Cl.: C12Q 1/68

(54) **VERWENDUNG EINER GENVERÄNDERUNG IM HUMANEN GNAS-GEN ZUR VORHERSAGE VON ERKRANKUNGSRISIKEN, KRANKHEITSVERLÄUFEN UND ZUR VORHERSAGE DES ANSPRECHENS AUF KRANKHEITSTHERAPIEN**
USE OF A GENE MUTATION IN THE HUMAN GNAS GENE FOR PREDICTING RISKS OF DISEASES, COURSES OF THE DISEASE AND FOR PREDICTING THE RESPONSE TO DISEASE THERAPIES
UTILISATION D'UNE MODIFICATION GENETIQUE DANS LE GENE GNAS HUMAIN POUR LA PREVISION DE RISQUES PATHOLOGIQUES ET DE DEROULEMENTS PATHOLOGIQUES ET LA PREVISION DE LA REACTION PAR RAPPORT A DES THERAPIES

(30) Priorität: 19.02.2003 DE 10307114; 20.10.2003 DE 10348600
(43) Veröffentlichungstag der Anmeldung: 16.11.2005
(73) Patentinhaber: Universität Duisburg-Essen, 45145 Essen (DE)
(72) Erfinder: FREY, Ulrich, 45130 Essen (DE); SIFFERT, Winfried, 45147 Essen (DE)
(74) Vertreter: Minderop, Ralph H.
(86) Internationale Anmeldenummer: PCT/EP2004/001563
(87) Internationale Veröffentlichungsnummer: WO 2004/076690

(56) Entgegenhaltungen:
- LIM SHARON H M ET AL.: "Mutational analysis of the GNAS1 exons encoding the stimulatory G protein in five patients with pseudohypoparathyroidism type 1a" JOURNAL OF PEDIATRIC ENDOCRINOLOGY AND METABOLISM, vol. 15, no. 3, March 2002 (2002-03), pages 259-268, XP008038540
- ALDRED M A ET AL: "ACTIVATING AND INACTIVATING MUTATIONS IN THE HUMAN GNAS1 GENE" HUMAN MUTATION, WILEY-LISS, NEW YORK, NY, US, vol. 16, no. 3, 2000, pages 183-189, XP008038624 ISSN: 1059-7794
- BASTEPE M ET AL: "POSITIONAL DISSOCIATION BETWEEN THE GENETIC MUTATION RESPONSIBLE FOR PSEUDOHYPOPARATHYROIDISM TYPE IB AND THE ASSOCIATED METHYLATION DEFECT AT EXON A/B: EVIDENCE FOR A LONG-RANGE REGULATORY ELEMENT WITHIN THE IMPRINTED GNAS1 LOCUS" HUMAN MOLECULAR GENETICS, OXFORD UNIVERSITY PRESS, SURREY, GB, vol. 10, no. 12, 1 June 2001 (2001-06-01), pages 1231-1241, XP008038583 ISSN: 0964-6906
- KIM S - J ET AL.: "Deletion polymorphism in the coding region of the human NESP55 alternative transcript of GNAS1" MOLECULAR AND CELLULAR PROBES, vol. 14, 2000, pages 191-194, XP004435425

## Beschreibung

Die Erfindung betrifft die Verwendung einer genomischen Genveränderung im Gen für die Gαs Untereinheit menschlicher G-Proteins, die durch das Gen GNAS (bzw. GNAS1) kodiert wird, zur Vorhersage von Krankheitsrisiken, Krankheitsverläufen und von Ansprechen auf Krankheitstherapien mittels pharmakologischer und nicht-pharmakologischer Maßnahmen und zur Vorhersage unerwünschter Arzneimittelwirkungen. Ferner betrifft die Erfindung die Bereitstellung von einzelnen Genveränderungen und Haplotypen, mit deren Hilfe weitere für die oben genannten Zwecke verwendbaren Genveränderungen detektiert und validiert werden können. Solche Genveränderungen können darin bestehen, dass im Exon 5 an Position 393 eine Substitution von Thymin durch Cytosin vorliegt, dass an Position -1211 eine Substitution von Guanin durch Adenin vorliegt, dass an Position -839 eine Substitution von Thymin durch Guanin vorliegt, oder dass an Position 2291 eine Substitution von Thymin durch Cytosin vorliegt. Die Genveränderuhgen können einzeln oder in beliebiger Kombination mittels dem Fachmann geläufigen Verfahren detektiert werden.

### Funktion und Bedeutung heterotrimerer G-Proteine

Alle Zellen des menschlichen Körpers verfügen über Membranrezeptoren an ihrer Oberfläche, über die alle Zellfunktionen gesteuert werden. Zu solchen Rezeptoren gehören die so genannten heptahelikalen Rezeptoren für Hormone, Neurotransmitter und Chemokine. Daneben gibt es eine Vielzahl von Rezeptoren für Wachstumsfaktoren und Rezeptoren mit intrinsischer Tyrosinkinaseaktivität, beispielsweise Rezeptoren für Insulin, Insulin-like Growth Factor, Epidermal Growth Factor, Platelet-derived Growth Factor und viele mehr.

Weiterhin existieren viele Rezeptoren, die für die Regulation der Blutbildung verantwortlich sind, wie z.B. der Rezeptor für Erythropoietin. Über solche Rezeptoren werden unter anderem Zellwachstum, Motilität, Genexpression, Apoptose und Chemotaxis gesteuert. Die genannten Rezeptoren übermitteln ihre Signale ins Zellinnere über die Aktivierung sog. heterotrimerer G-Proteine. Diese G-Proteine bestehen aus einer großen Familie unterschiedlicher Isoformen und sind jeweils aus unterschiedlichen α-, β- und γ-Untereinheiten zusammengesetzt. Derzeit sind 5 β-Untereinheiten, 13 γ-Untereinheiten und mehr als 20 α-Untereinheiten bekannt, die durch unterschiedliche Gene kodiert werden (Farfel Z et al. The expanding spectrum of G protein diseases. N Engl J Med. 1999 Apr 1;340(13):1012-20).

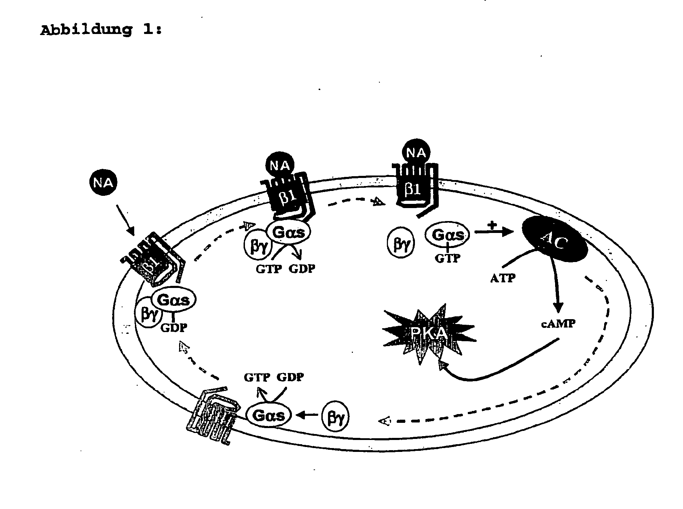

Durch die Kombination aus diesen verschiedenen α-, β- und γ-Untereinheiten entsteht eine Vielzahl unterschiedlicher heterotrimerer G-Proteine. Dabei determiniert die Isoform- Kombination, welches Heterotrimer durch einen bestimmten Rezeptor aktiviert werden kann. Die βγ-Untereinheiten sind funktionell als Monomer zu betrachten. Im Ruhezustand hat die α-Untereinheit GDP gebunden (Abbildung 1; Legende: NA, Noradrenalin; β1, β1-adrenerger Rezeptor; Gαs, stimulatorische α-Untereinheit, AC, Adenylylcyclase; PKA, Proteinkinase A;). Nach Aktivierung eines koppelnden Rezeptors setzt die α-Untereinheit GDP im Austausch gegen GTP frei und es kommt zur Dissoziation der βγ-Untereinheiten von den α-Untereinheiten. Sowohl die freien α- als auch die βγ-Untereinheiten können die Aktivität einer Vielzahl unterschiedlicher Effektoren steuern. Hierzu gehören beispielsweise Ionenkanäle, die Adenylylzyklase, die PI3-Kinase, unterschiedliche MAP-Kinasen usw. Die α-Untereinheiten verfügen über eine intrinsische GTPase-Aktivität die das nach Aktivierung gebundene GTP zu GDP hydrolysiert. Nachfolgend re-assoziieren die freigesetzten βγ-Untereinheiten wieder mit der α-Untereinheit, wodurch der Aktivierungszyklus beendet wird. Damit steht das Heterotrimer für einen erneuten Aktivierungszyklus zur Verfügung (Bourne HR. How receptors talk to trimeric G proteins. Curr Opin Cell Biol. 1997;9(2):134-42). Ein Schema des G-Proteinzyklus ist in Abbildung 1 dargestellt. Die Aktivierung solcher G-Proteine ist der entscheidende Schritt für die Zellaktivierung. Aufgrund der überragenden Bedeutung von G-Proteinen ist es unmittelbar einleuchtend, dass Mutationen oder genetische Polymorphismen in Genen, die für G-Proteine kodieren, einen nachhaltigen Einfluss auf die Aktivierbarkeit von Zellen haben müssen, falls diese Mutationen die Funktion oder Expression von G-Protein-Untereinheiten beeinflussen. Damit werden auch Erkrankungsrisiken oder Krankheitsverläufe in entscheidender Weise beeinflusst. Zudem ist das Ansprechen auf die Therapie von Erkrankungen, sei es durch Pharmaka oder durch andere Maßnahmen wie Bestrahlung, Diäten, Operationen, invasive Eingriffe etc. von der Aktivierbarkeit von G-Proteinen abhängig.

### Bedeutung der Gas-Untereinheit

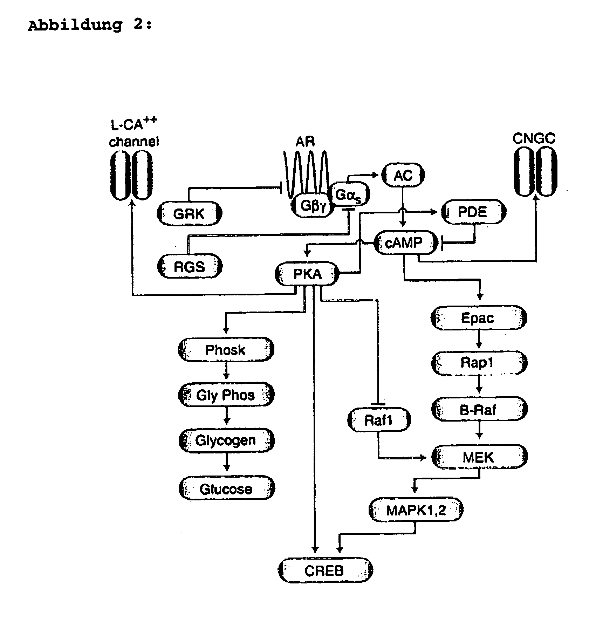

Die Gas-Untereinheit wird in allen Körperzellen des Menschen exprimiert. Ihre Stimulation führt u.a. zur Aktivierung der Adenylyzyklase und damit zu einem Anstieg der intrazellulären cAMP-Konzentration. Damit können z. B. cAMP-abhängige Proteinkinasen aktiviert werden. Aber auch andere cAMP-abhängige Signalkaskaden werden durch Stimulation von Rezeptoren, die an Gαs koppeln gehemmt oder aktiviert. Ferner kann Gαs die Aktivität von Ionenkanälen, z.B. von Kalium- oder Kalzium-Kanälen, regulieren (siehe Abbildung 2). Das Diagramm in Abbildung 2 zeigt wie demonstrates cAMP-Weg mit vielfältigen Signaltransduktionskomponenten verbunden ist, einschließlich Ionenkanäle, Transkriptionsfaktoren und metabolische Enzyme. AC, Adenylyl cyclase; PKA, Protein Kinase A; PDE, Phosphodiesterase; L-Ca⁺⁺ Kanal, L-Typ Ca2⁺-Kanal; CNGC, cyclic nucleotide-gated channel; PhosK, Phosphorylase-Kinase; GlyPhos, Glycogen Phosphorylase; CREB, cAMP response element-binding protein; EPAC, the cAMP- and AMP-regulated exchange factor for Rap1; Rap1, eine kleine GTPase; MAPK, mitogen-activated protein kinase; Raf1 und B-Raf, MAP kinase kinase kinasen; MEK, MAPK/ERK-Kinase; MEKK, MAPK/ERK-Kinase-Kinase; GRK, G-Protein-Rezeptorkinase; RGS, "regulators of G protein signaling"; βAR, β-adrenerger Rezeptor (aus: Neves at al, G protein pathways.Science. 2002 May 31;296(5573):1636-9). Eine Vielzahl von Rezeptoren koppeln an Gαs, z.B. die Rezeptoren für Adenosin, Adrenalin, Noradrenalin, P2-purinerge Rezeptoren, Opioide, Dopamin, Epidermal Growth Factor, FSH, VIP, Thyroliberin, Glucagon, Vasopressin, Histamin und viele mehr. Nach Stimulation Gasgekoppelter Rezeptoren wird in vielen Zelltypen Apoptose induziert, so dass sich ein Zusammenhang zu Tumorerkrankungen und deren Verlauf und Therapieansprechen, aber auch ein Zusammenhang zu entzündlichen Erkrankungen und deren Verlauf und Therapieansprechen ergibt. Daneben werden vielfältige Stoffwechselwege durch Gαs reguliert. Eine Hemmung der Expression von Gαs in Fibroblasten beschleunigt deren Differenzierung zu Adipozyten (Wang Hy et al., Antisense oligodeoxynucleotides to GS protein alpha-subunit sequence accelerate differentiation of fibroblasts to adipocytes. Nature. 1992 Jul 23;358(6384):334-7; Wang H et al. G(s)alpha repression of adipogenesis via Syk.J Biol Chem. 1999 Nov 5;274(45):32159-66; Übersicht bei Neves at al, G protein pathways.Science. 2002 May 31;296(5573):1636-9).

### Das GNAS Gen

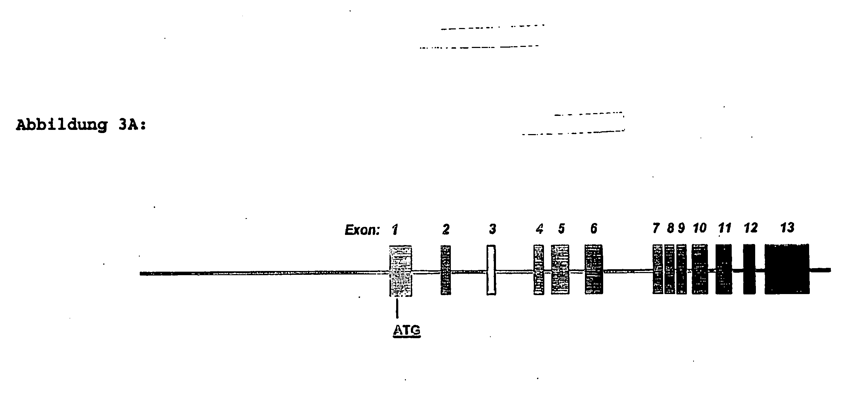

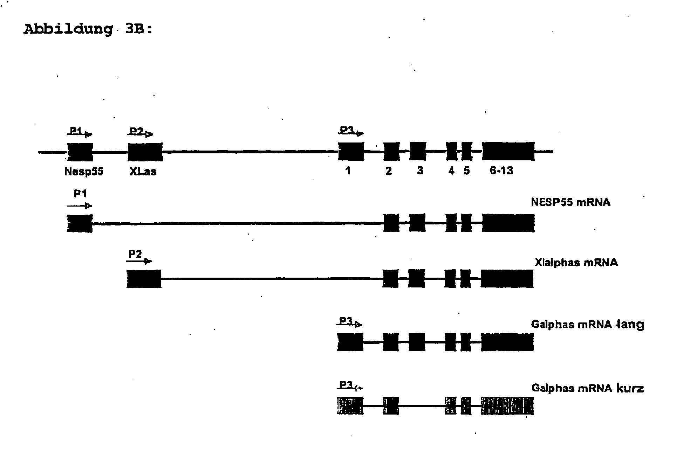

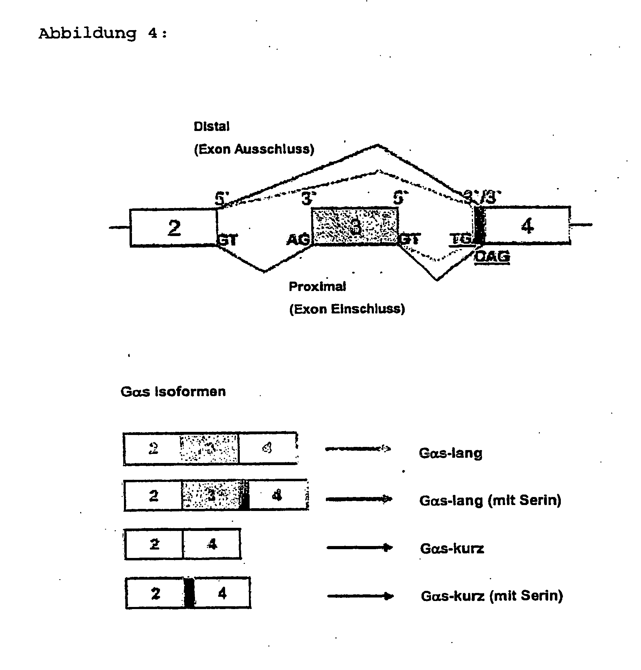

Das humane Gαs Gen (GNAS) ist auf Chromosom 20q13.2-13.3 lokalisiert. An dieser Stelle soll darauf hingewiesen werden, dass in der Literatur die Bezeichnungen "GNAS" und "GNAS1" synonym als Bezeichnung für das genannte Gen verwandt werden. Wir verwenden hier die Bezeichnung "GNAS". Eine schematische Darstellung der Intron/Exon-Struktur des humanen GNAS findet sich in Abbildung 3A. Die Benutzung unterschiedlicher Promotoren, P1, P2 oder P3 führt zur Entstehung unterschiedlicher mRNAs und damit zu unterschiedlichen Genprodukten (prä-mRNAS), vgl. Abbildung 3B. Zwei lange (Gαs-1 und -2) und zwei kurze (Gαs-3 und -4) Isoformen von Gαs resultieren aus alternativem Spleißen von Exon 3 (T. Kozasa et al. Isolation and characterization of the human Gs alpha gene. Proc. Natl. Acad. Sci. U. S. A 85: 2081-2085, 1988.). Die Verwendung einer alternativen Spleiß-Acceptor Site für Exon 4 führt zur Insertion eines weiteren Serins in Gₛα-2 and -4 (Abbildung 4; P. Bray et al. Human cDNA clones for four species of G alpha s signal transduction protein. Proc.Natl.Acad.Sci.U.S.A. 83 (23):8893-8897, 1986). Abbildung 4 zeigt unterschiedliche Spleißvarianten von Gαs. Die Benutzung unterschiedlicher Spleißsites führen zu einem Einschluß eines CAG-Triplets (Serin) aus Exon 4. Die Exklusion von Exon 3 führt zur Ausbildung von Gαs-kurz. Die Spleißvarianten unterscheiden sich bezüglich ihrer Gewebeverteilung, ihrer Aktivierbarkeit durch G-Protein gekoppelte Rezeptoren und ihrer Aktivierung von Effektoren (J. Novotny and P. Svoboda. The long (Gs(alpha)-L) and short (Gs(alpha)-S) variants of the stimulatory guanine nucleotide-binding protein. Do they behave in an identical way? J.Mol.Endocrinol. 20 (2):163-173, 1998; R. Seifert et al. Different effects of Gsalpha splice variants on beta2-adrenoreceptor-mediated signaling. The Beta2-adrenoreceptor coupled to the long splice variant of Gsalpha has properties of a constitutively active receptor. J.Biol.Chem. 273 (18):5109-5116, 1998).

### Somatische Mutationen in GNAS

Es wurden eine Reihe von somatischen Mutationen in GNAS-Gen beschrieben, die zu seltenen Stoffwechselerkrankungen beitragen. Meistens handelt es sich hierbei um aktivierende (Endokrine GH-sezernierende Tumore, McCune-Albright-Syndrom, Fibröse Knochendysplasie) oder inaktivierende (z.B. Pseudohypoparathyraodismus Typ IA, Pseudopseudohypoparathyroidismus, (PPHP)) Mutationen im Bereich der GTPase-Region des Protein. (Übersicht bei: Weinstein et al., Endocrine Manifestations of Stimulatory G Protein α-Subunit Mutations and the Role of Genomic Imprinting Endocrine Reviews 22 (5): 675-705, 2001). Im Unterschied zu single nucleotide polymorphismen (SNP's) werden diese Mutationen bei den entsprechenden Patienten beispielsweise nicht in peripheren Blutzellen gefunden.

### Genomische Mutation in GNAS

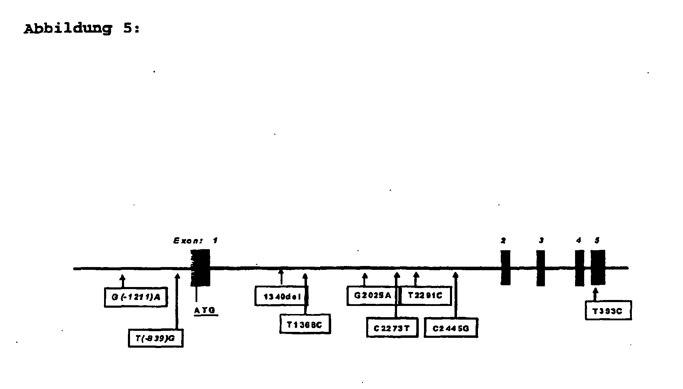

Jia et al. untersuchten 1999, ob das GNAS Gen zum Risiko einer essentiellen Hypertonie beiträgt (Jia H. et al., Association of the Gsα Gene With Essential Hypertension and Response to β-Blockade Hypertension. 1999;34:8-14.). Sie untersuchten einen häufigen stummen T393C Polymorphismus (SNP, single nucleotide polymorphism)in Exon 5 des GNAS-Gens (Numerierung entsprechend der Sequenz der cDNA; Abbildung 5). Dieser Polymorphismus ist durch das Fehlen einer Restriktionsschnittstelle für die Endonuklease FokI (Fok- beim T-Allel) oder die Anwesenheit dieser Schnittstelle (Fok+ beim C-Allel) gekennzeichnet. Das T393-Allel fand sich häufiger bei Hypertonikern als bei normotensiven Kontrollen. Dieser Befund wurde später durch andere Untersucher bestätigt (Abe M et al., Association of GNAS gene variant with hypertension depending on smoking status. Hypertension. 2002;40:261-5). Daneben wurde beschrieben, dass hypertensive 393C-Allel-Träger bezüglich der Blutdrucksenkung besser auf β-Blocker ansprechen als T393-Allelträger Jia H. et al., Association of the Gsα Gene With Essential Hypertension and Response to β-Blockade Hypertension. 1999;34:8-14.). Andere Untersucher zeigten einen Zusammenhang zwischen dem T3 93C-Status und dem Risiko für eine orthostatische Hypotonie (Tabara, Y. et al., Polymorphisms of genes encoding components of the sympathetic nervous system but not the renin+angiotensin system as risk factors for orthostatic hypotension Journal of Hypertension-2002, 20:651 -656). Damit ist gezeigt, dass der T393C-Status bislang nur mit der Blutdruckregulation bzw. dem Ansprechen auf eine ß-Adrenozeptorblockade in Verbindung gebracht wurde. Eine Korrelation zu anderen Erkrankungen, Erkrankungsverläufen oder zum Ansprechen auf Pharmaka im allgemeinen wurde bislang nicht beschrieben. Da dieser T3 93C-Nukleotidaustausch stumm ist und die kodierte Aminosäure (Isoleucin) nicht verändert, muss es weitere Polymorphismen geben, die mit dem T393C-Polymorphismus im Kopplungsungleichgewicht stehen.

Das Journal of Pediatric Endocrinology & Metabolism 15, 259-268 (2002) berichtet, dass die Analyse des GNAS1-Gens im Hinblick auf Mutationen ein starkes unterstützendes Werkzeug in der Diagnose von Pseudohypoparathyreoidismus (PHPla) sei. Es ist von drei neuen Mutationen in D1 die Rede. HUMAN MUTATION 16:183-189 (2000) beschreibt das GNAS1-Gen aktivierende und inaktivierende Mutationen. Hier geht es darum, dass verschiedene Mutationen dieses Gens die Aktivität des Gens beeinflussen; es geht nicht um das Heranziehen einer bestimmten Mutation zur Beurteilung eines spezifischen Risikos für eine Krankheit. Human Molecular Genetics 2001, Vol.10, No.12 1231-1241 bringt den Pseudohypoparathyreoidismus mit einem veränderten Methylierungsmuster im Exon A/B des GNAS1-Gen in Verbindung. Molecular and Cellular Probes (2000) 14, 191-194 beschäftigt sich mit Mutationen des NESP55-Transkripts von GNAS1 und berichtet über das Auffinden von 6 SNIPs und einer Deletion. Der Deletion kann kein bestimmter Phänotyp zugeordnet werden.

### Aufgabe der Erfindung

Der hier beschriebenen Erfindung liegt die Aufgabe zu Grunde:
a. funktionsverändernde genomische Polymorphismen und Haplotypen im Gen GNAS bereitzustellen, die entweder zu einem Aminosäurenaustausch führen, oder
b. die das Spleißverhalten beeinflussen, oder
c. die zur Änderung der Proteinexpression oder zur Änderung der Expression von Spleißvarianten führen, oder
d. die zum Auffinden und/oder Validieren weiterer Polymorphismen bzw. Haplotypen im Gen GNAS geeignet sind;
e. Nukleotidaustausche und Haplotypen bereitzustellen, die geeignet sind, generell Krankheitsrisiken- und Verläufe vorherzusagen;
f. Nukleotidaustausche und Haplotypen bereitzustellen, die geeignet sind, generell Ansprechen auf Pharmaka und Nebenwirkungen vorherzusagen;
g. Nukleotidaustausche und Haplotypen bereitzustellen, die geeignet sind, generell die Wirkung anderer Therapieformen vorherzusagen (Bestrahlung; Wärme, Hitze, Kälte, Bewegung etc.

Wegen der grundlegenden Bedeutung von Gαs für die Signaltransduktion sind solche Polymorphismen bzw.. Haplotypen geeignet, generell Erkrankungsrisiken bzw. Krankheitsverläufe bei allen Erkrankungen vorherzusagen bzw. Therapieansprechen/Therapieversagen oder unerwünschte Nebenwirkungen für alle Pharmaka oder nichtpharmakologische Therapien vorherzusagen.

### Nachweis neuer Polymorphismen im Gen GNAS vor Exon 1 und im Intron 1

Hauptgegenstand der vorliegenden Erfindung ist das Auffinden der vor Exon 1 liegenden Polymorphismen G(-1211)A und T(-839)G und der im Intron 1 liegenden Polymorphismen 1340del, T1368C, G2025A, C2273T, T2291C und C2445G (Abbildung 5), die durch systematische Sequenzierung von DNAs von Menschen, die homozygot für das 393C-Allel oder homozygot für das T393-Allel sind, gefunden wurden. Hierzu wurden Gensequenzen, die vor Exon1 oder im Intron 1 von GNAS liegen, mittels PCR-Reaktion amplifiziert und gemäß der Methode nach Sanger sequenziert. Die dazu erforderlichen Verfahren, z.B. das Ableiten von für die PCR-Reaktion erforderlichen Primerpaaren und die Auswahl von Sequenzier-Primern sind dem Fachmann geläufig. Hierbei wurden neue Polymorphismen gefunden, wobei in der Promotorregion an Position -1211 eine Substitution von Guanin durch Adenin vorliegt (G(-1211)A-Polymorphismus), oder wobei an Position -839 eine Substitution von Thymin durch Guanin vorliegt (T(-893)G-Polymorphismus). Im Intron 1 wurden Polymorphismen mit folgenden Substitutionen identifiziert: an Position 1340 erfolgt eine Deletion von 12 Thymidinen, an 1368 liegt eine Substitution von Thymidin durch Cytosin vor (T1368), an 2025 erfolgt ein Austausch von Guanin durch Adenin (G2025A), an 2273 liegt eine Substitution von Thymin durch Cytosin vor (C2273T), an 2291 liegt ein Austausch von Thymidin durch Cytosin vor (T2291C) und an 2445 existiert eine Substitution von Cytosin durch Guanin (C2445G). Die Nummerierung dieser SNPs erfolgt in der Weise, dass dem Nukleotid A des Startcodon ATG die Nummer +1 zugeordnet wird. Da es der Konvention entsprechend die Nummer 0 nicht gibt, ist dem vor dem A des Startcodon ATG liegenden Nukleotid die Nummer -1 zugeordnet.

Der Nachweis dieser SNPs im Sinne ihrer erfindungsgemäßen Verwendung kann mit beliebigen, dem Fachmann geläufigen Verfahren nachgewiesen werden, z.B. direkte Sequenzierung, PCR mit nachfolgender Restriktionsanalyse, reverse Hybridisierung, Dot-blot- oder slot-blot-Verfahren, Massenspektrometrie, Taqman®- oder Light-Cycler®-Technologie, Pyrosequencing®. Invader®-Technologie, Luminex-Verfahren etc. Ferner können diese Genpolymorphismen gleichzeitig nach Mulitplex-PCR und Hybridisierung an ein DNA-Chip detektiert werden.

Verteilung der T393C, G(-1211)A und T(-839)G Polymorphismen und abgeleiteter Genotypen bei unterschiedlichen Ethnien, Nachweis von Haplotypen und Verwendung dieser Genotypen zum Auffinden weiterer relevanter Polymorphismen und Haplotypen.

Hierzu wurden unterschiedliche DNA-Proben von Kaukasiern, Schwarzafrikanern und Chinesen genotypisiert. Das Ergebnis ist in folgenden Tabellen dargestellt

| GNAS T393C | | | |
|---|---|---|---|
| Genotyp | Kaukasier | Schwarzafrikaner | Chinesen |
| CC, (n,%) | 49 (25,1) | 6 (6,0) | 12 (10,8) |
| CT, (n,%) | 97 (49,7) | 32 (32,0) | 53 (47,7) |
| TT (n,%) | 49 (25,1) | 62 (62,0) | 46 (41, 4) |
| Summe | 195 | 100 | 111 |
| % T | 50,0 % | 78,0% | 65,3% |

Diese Genotypverteilung ist im chi² test mit einem Chi 43,3 und einem P<0.0001 hochsignifikant verschieden. Die T393-Allelfrequenz (%T) ist bei Schwarzafrikanern am höchsten.

| GNAS G(-1211)A | | | |
|---|---|---|---|
| Genotyp | Kaukasier | Schwarzafrikaner | Chinesen |
| AA, (n,%) | 26 (13,3) | 0 (0,0) | 47 (47,5) |
| GA, (n,%) | 104 (53,3) | 0 (0,0) | 36 (36,4) |
| GG (n, %) | 65 (33,3) | 100 (100,0) | 16 (16,2) |
| Summe | 195 | 100 | 99 |
| % G | 60,0 % | 100 % | 34,3 % |

Diese Genotypverteilung ist im chi² test mit einem Chi 208,30 und einem P<0.0001 hochsignifikant verschieden. Die (-1211)G-Allelfrequenz (%G) ist bei Schwarzafrikanern am höchsten, gefolgt von Kaukasiern und Chinesen.

| GNAS T(-839)G | | | |
|---|---|---|---|
| Genotyp | Kaukasier | Schwarzafrikaner | Chinesen |
| GG, (n,%) | 10 (5,1) | 0 (0,0) | 0 (0) |
| TG, (n,%) | 52 (26,7) | 1 (1,0) | 0 (0) |
| TT (n, %) | 133 (68,2) | 99 (99,0) | 100 (100) |
| Summe | 195 | 100 | 99 |
| % T | 81,5 % | 99,5 % | 100 % |

Diese Genotypverteilung ist im chi² test mit einem Chi 72.2 und einem P<0.0001 hochsignifikant verschieden. Die (-839)T-Allelfrequenz (%T) ist bei Schwarzafrikanern und Chinesen am höchsten. Aus diesen Verteilungen kann man folgern, dass entwicklungsgeschichtlich (bezogen auf Kaukasier) das T393-Allel, das (-1211)G-Allel und das (-893)G-Allel die jeweiligen."Urzustände" darstellen. Solche Unterschiede der Genotypverteilung bei unterschiedlichen Ethnien weisen in der Regel darauf hin, dass assoziierte Phänotypen für die Evolution bedeutsam waren und den Trägern einen bestimmten Vorteil brachten. Es ist dem Fachmann bekannt, dass ethnisch unterschiedliche Genotypverteilung ein Hinweis darauf sind, dass auch heute noch bestimmte Genotypen- und Haplotypen mit bestimmten Erkrankungen oder physiologischen und pathopyhsiologischen Reaktionsweisen oder Ansprechen auf Therapie, z.B. mit Pharmaka, assoziiert sind.

Eine weitere Analyse zeigt ein Kopplungsungleichgewicht zwischen den drei Polymorphismen bei Kaukasiern. Unter Kopplungsungleichgewicht versteht man das Auftreten von Allelkombinationen (Haplotypen), die statistisch eindeutig häufiger oder seltener gemeinsam vorkommen, als dies bezogen auf ihre Frequenz zu erwarten wäre.

Die folgenden Tabelle zeigt für Kaukasier die Verteilung von T393C Genotypen stratifiziert nach GNAS G(-1211)A Genotypen.

| | GNAS G(-1211)A | | | |
|---|---|---|---|---|
| GNAS T393C | AA | GA | GG | Gesamt |
| CC | 2 | 25 | 22 | 49 |
| CT | 11 | 53 | 33 | 97 |
| TT | 13 | 26 | 10 | 49 |
| Gesamt | 26 | 104 | 65 | 195 |

Man erkennt eine deutliche Akkumulation des (-1211)G-Allels bei Trägern eines T393-Allels (p<0.01).Die folgenden Tabelle zeigt für Kaukasier die Verteilung von T393C Genotypen stratifiziert nach GNAS T(-893)G - Genotypen.

| | GNAS T(-893)G | | | |
|---|---|---|---|---|
| GNAS T393C | GG | TG | TT | Gesamt |
| CC | 5 | 16 | 28 | 49 |
| CT | 4 | 16 | 67 | 97 |
| TT | 1 | 20 | 38 | 49 |
| Gesamt | 10 | 52 | 133 | 195 |

Man erkennt eine Akkumulation des (-893)T-Allels bei Trägern eines T393-Allels. Die folgenden Tabelle zeigt für Kaukasier die Verteilung von G(-1211)A-Genotypen stratifiziert nach GNAS T(-893)G -Genotypen.

| | GNAS T(-893)G | | | |
|---|---|---|---|---|
| GNAS G(-1211)A | GG | TG | TT | Gesamt |
| AA | 0 | 0 | 26 | 26 |
| GA | 0 | 32 | 72 | 104 |
| GG | 10 | 20 | 35 | 65 |
| Gesamt | 10 | 52 | 133 | 195 |

Man erkennt eine deutliche Akkumulation des (-893)T-Allels bei Trägern eines -1211G-Allels (p<0.0001).

Weitergehende Analysen zeigen dass bei Kaukasiern präferentiell die Haplotypen T393 + G(-1211) + T(-839) (zu 60 %) und 393C + (-1211)A + (-839)G zu 47 % vorkommen.Daneben sind alle denkbaren Permutationen gefunden worden.

### Kopplungsungleichgewicht der Intron1 Polymorphismen zu G(-1211)A und T393C

Bei hundert genotypisierten Kaukasiern wurde das Kopplungsungleichgewicht der Intron1 Polymorphismen untereinander sowie das Kopplungsungleichgewicht der Intron 1 Polymorphismen zu dem G(-1211)A und T393C Polymorphismus errechnet. Die Zählung und Zuordnung der entsprechenden Nukleotidpositionen erfolgt in der Weise, dass dem Startcodon ATG in Exon 1 die Position +1 zugeordnet wird (Siehe Abbildung 5). Abbildung 5 zeigt Struktur von GNAS und Lage neuer und bekannter Polymorphismen. Dargestellt sind der T393C-Polymorphismus in Exon 5, sowie die Polymorphismen G(-1211)A und T(-839G) vor dem Exon 1. ATG bezeichnet das Startcodon in Exon 1. Die Nummerierung der Promoter/Enhancerbereich - Polymorphismen und des Intron 1 Bereiches erfolgt, wie bereits erwähnt, in der Weise, dass dem Nukleotid A des Startcodons ATG in Exon 1 der Wert +1 zugeordnet und dann über die Exon-Introngrenze weitergezählt wird. Die Nomenklatur des T393C Polymorphismus in Exon 5 entspricht der Nummerierung in der cDNA. Von dort aus werden die Nukleotide über die Intron-Exongrenze hinaus fortlaufen in das Intron1 hineingezählt.

Die folgenden Tabelle zeigt für Kaukasier die Verteilung von C2273T Genotypen stratifiziert nach GNAS A2025G Genotypen.

| | | C2273T | | | Gesamt |
|---|---|---|---|---|---|
| | | CC | CT | TT | |
| A2025G | AA | | | 16 | 16 |
| | AG | | 52 | | 52 |
| | GG | 32 | | | 32 |
| Gesamt | | 32 | 52 | 16 | 100 |

Man erkennt eine komplette Kopplung der Genotypen der beiden Polymorphismen. Zur weiteren Analyse wird daher nur der C2273T Polymorphismus betrachtet.

Die folgenden Tabelle zeigt für Kaukasier die Verteilung von T1368C und T2291C Genotypen stratifiziert nach GNAS C2445G Genotypen.

| | | | T2291C | | | Gesamt |
|---|---|---|---|---|---|---|
| C2445G | | | CC | CT | TT | |
| CC | T1368C | CC | | | 10 | 10 |
| CG | T1368C | TC | | 41 | | 41 |
| GG | T1368C | TT | 49 | | | 49 |
| | Gesamt | | 49 | 41 | 10 | 100 |

Man erkennt eine komplette Kopplung der Genotypen der drei Polymorphismen. Zur weiteren Analyse wird daher nur der T2291C Polymorphismus betrachtet.

Die folgenden Tabelle zeigt für Kaukasier die Verteilung von C2273T Genotypen stratifiziert nach GNAS 1340del Genotypen. Das "D" steht für die Deletion von zwölf Thymidinresten.

| | | D1340I | | | Gesamt |
|---|---|---|---|---|---|
| | | DD | ID | II | |
| C2273T | CC | | 5 | 27 | 32 |
| | CT | | 49 | | 49 |
| | TT | 16 | | | 16 |
| Gesamt | | 16 | 54 | 27 | 97 |

Man erkennt eine fast komplette Kopplung der Genotypen der beiden Polymorphismen mit einem Chi-Quadrat Wert von 171.2 (p<0.00001). Da auch hier eine nahezu hundertprozentige Kopplung der beiden Polymorphismen vorliegt, wird im weiteren weiterhin der C2273T betrachtet.

Die folgenden Tabelle zeigt für Kaukasier die Verteilung von C2273T Genotypen stratifiziert nach GNAS G(-1211)A Genotypen.

| | | G(-1211)A | | | Gesamt |
|---|---|---|---|---|---|
| | | AA | GA | GG | |
| C2273T | CC | | | 32 | 32 |
| | CT | | 52 | | 52 |
| | TT | 16 | | | 16 |
| Gesamt | | 16 | 52 | 32 | 100 |

Man erkennt eine komplette Kopplung der Genotypen der beiden. polymorphismen. Zur weiteren Analyse wird daher nur der G(-1211)A Polymorphismus betrachtet.

Die folgenden Tabelle zeigt für Kaukasier die Verteilung von G(-1211)A Genotypen stratifiziert nach GNAS T2291C Genotypen.

| | | T2291C | | | Gesamt |
|---|---|---|---|---|---|
| | | CC | TC | TT | |
| G(-1211)A | GG | 7 | 15 | 10 | 32 |
| | GA | 26 | 26 | | 52 |
| | AA | 16 | | | 16 |
| Gesamt | | 49 | 41 | 10 | 100 |

Man erkennt eine deutliche Akkumulation des 2291T-Allels bei Trägern eines -1211G-Allels (Chi-Quadrat=42.4, p<0. 0001).

Die folgenden Tabelle zeigt für Kaukasier die Verteilung von T393C Genotypen stratifiziert nach GNAS T2291C Genotvoen.

| | | T2291C | | | Gesamt |
|---|---|---|---|---|---|
| | | CC | TC | TT | |
| T393C | CC | 6 | 11 | 7 | 24 |
| | CT | 21 | 25 | 2 | 48 |
| | TT | 22 | 5 | 1 | 28 |
| Gesamt | | 49 | 41 | 10 | 100 |

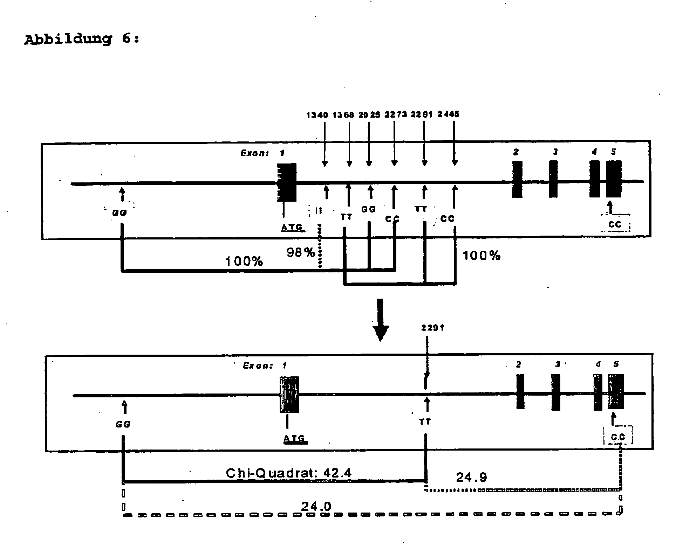

Man erkennt eine Akkumulation des 2291C-Allels bei Trägern eines T393-Allels (Chi-Quadrat=24.9, p<0.0001 ). Abbildung 6 zeigt Kopplungsungleichgewichte zwischen Genotypen der Intron 1 Polymorphismen und G(-1211)A sowie T393C. Polymorphismen, die zu >=98% aneinander koppeln, werden zur Vereinfachung zusammengefasst. Die Güte der Kopplung wird durch den Chi-Quadrat wird gekennzeichnet. Dabei spricht man bei einem Freiheitsgrad von 3 bei einem Wert von >7.82 von einer signifikanten Kopplung. Je größer dieser Wert ist, desto enger das Kopplungsungleichgewicht.

Weitergehende Analysen zeigen dass bei Kaukasiern präferentiell die Haplotypen T393 + (-1211)A + 2291C (zu 58%) und 393C + G(-1211) + T2291 zu 45 % vorkommen.

Daneben sind alle denkbaren Permutationen gefunden worden.

Ein Gegenstand der Erfindung ist es, dass diese neuen Polymorphismen und Haplotypen dazu benutzt werden können um weitere relevante genomische Genveränderungen in GNAS oder benachbarten Genen zu detektieren und zu validieren, die z.B. mit Genotypen im Gen GNAS im Kopplungsungleichgewicht stehen. Dies können auch Gene sein, die ebenfalls auf Chromosom 20 liegen, aber in großer Entfernung vom Gen GNAS. Hierzu wird folgendermaßen vorgegangen:
1. Für bestimmte Phänotypen (zelluläre Eigenschaften, Krankheitszustände, Krankheitsverläufe, Arzneimittelansprechen usw.) wird zunächst eine Assoziation mit den Polymorphismen T393C, G(-1211)A, T2291C und T(-839)G und den Genotypen in Intron1 hergestellt, wobei diese Assoziationen für jeden Genotyp einzeln oder unter Verwendung aller Permutationen der Haplotypen ausgestellt werden können.
2. Für neu detektierte Genveränderungen in GNAS oder benachbarten Genen wird untersucht, ob bereits bestehende Assoziationen unter Verwendung oben beschriebener Geno- oder Haplotypen verstärkt oder abgeschwächt werden.

### Funktionelle Bedeutung des T393C Polymorphismus und daran koppelnder Polymorphismen

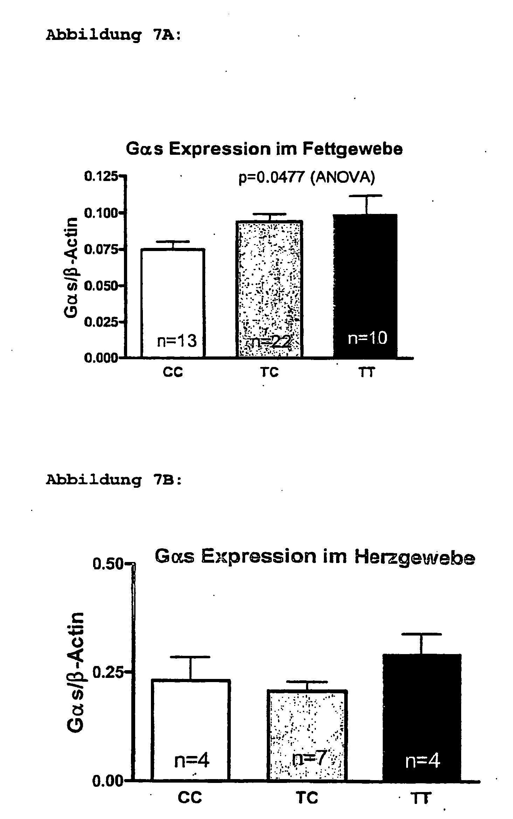

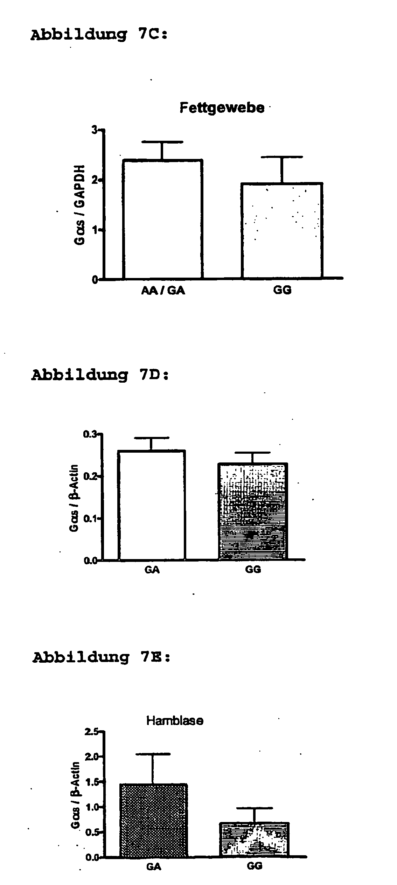

Es wurde untersucht, welche funktionellen Änderungen Genveränderungen im Gen GNAS zuzuordnen sind. Denkbar sind hier beispielsweise eine Korrelation zu alternativem Spleißen, gewebespezifiache Expression oder eine Überexpression des Gas -Proteins in Abhängigkeit von Genotypen des T393C-Polymorphismus bzw. des A-1211G-Polymorphismus. Hierzu wurde mRNA aus menschlichem Herz- und Fettgewebe gewonnen und mittels reverser Transkriptase in cDNA umgeschrieben. Das Verfahren ist dem Fachmann geläufig. Nachfolgend wurde das Expressionsniveau mittels Realtime-PCR (Taqman-Verfahren) bestimmt und mit dem Expressionsniveau des Housekeeping-Gens β-Actin abgeglichen. Die Ergebnisse sind in Abbildung 7 dargestellt. Abbildung 7A zeigt die relative Expression von Gαs mRNA (Ratio Gas/β-Actin)in humanem Fettgewebe in Abhängigkeit von Genotypen des T393C-Polymorphismus. Bei T-Allelträgern des T393C-Polymorphismus findet man signifikant höhere Expressionswerte als bei hömozygoten CC-Genotypen. In ähnlicher Weise findet man im humanen Herzgewebe bei TT-Genotypen relativ höhere Expressionswerte als bei CC- und TC-Genotypen des T393C-Polymorphismus (Abbildung 7B). Abbildung 7B zeigt die relative Expression von Gαs mRNA (Ratio Gas/β-Actin)in humanem Fettgewebe in Abhängigkeit von Genotypen des T393C-Polymoxphismus. Ähnliches gilt für die Analyse nach dem A(-1211)G- Polymorphismus. Bei A-Allelträgern findet man im Fettgewebe (Abbildung 7C; zeigt die relative Expression von Gαs mRNA (Ratio Gαs/GAPDH)in humanem Fettgewebe in Abhängigkeit von Genotypen des G(-1211)A-Polymorphismus), im Herzgewebe (Abbildung 7D; zeigt die relative Expression von Gαs mRNA (Ratio Gas/GAPDH)in humanem Herzgewebe in Abhängigkeit von Genotypen des G(-1211)A-Polymorphismus) und im Harnblasengewebe (Abbildung 7E; zeigt die relative Expression von Gαs mRNA (Ratio Gαs/GAPDH) in humanem Harnblasengewebe in Abhängigkeit von Genotypen des G(-1211)A-Polymorphismus)eine verstärkte Expression der Gαs mRNA, sowohl absolut, als auch in Relation zu Housekeeping-Genen (z.B. GAPDH, β-Aktin).

Damit wurde nachgewiesen, dass es im Gen GNAS Genveränderungen gibt, die eine Expressionsänderung von Gαs in unterschiedlichen Geweben bewirken. Dies kann der T393C-Polymorphismus, der A(-1211)G - Polymorphismus sein, der T2291C - Polymorphismus sein, oder Polymorphismen, die mit diesen im Kopplungsungleichgewicht stehen. Bestandteil der hier beschrieben Erfindung ist es damit auch, die Expression von Gαs auf mRNA-Ebene oder Proteinebene zu quantifizieren, mit bekannten Polymorphismen des GNAS zu assoziieren und neue, noch besser geeignete Polymorphismen zu entdecken und zu validieren.

Die hier gezeigten Befunde einer genotypabhängigen Expression von Gαs in menschlichen Geweben ist überaus bedeutsam. Bei transgenen Tieren führt die Überexpression von Gαs zur vermehrten Apoptose kardialer Myozyten (Geng Y. et al., Apoptosis of cardiac myocytes in Gsalpha transgenic mice. Circ Res. 1999, 84, 34-42) als Ursache einer Herzinsuffizienz. Bei solchen Tieren kommt es zu vermehrtem Ca2+-Einstrom in Myozyten (Kim SJ et al., Differential regulation of inotropy and lusitropy in overexpressed Gsalpha myocytes through cAMP and Ca2+ channel pathways. J Clin Invest. 1999 Apr;103(7):1089-97). Daneben zeigen diese Tiere eine erhöhte Herzfrequenz und einen erhöhten Blutdruck (Uechi M et al., Depressed heart rate variability and arterial baroreflex in conscious transgenic mice with overexpression of cardiac Gsalpha. Circ Res. 1998 Mar 9;82(4):416-23) Gleiche Effekte sind daher auch bei Menschen zu erwarten, die auf Grund einer Genveränderung in GNAS das Gas-Protein überexprimieren. Hier erwarten wir ein erhöhtes kardiovaskuläres Risiko. Dazu gehört ein erhöhtes Risiko für Adipositas, Hypertonie, Schlaganfall, koronare Herzkrankheit, Myokardinfarkt, Präeklampsie etc. Außerdem erwarten wir ein geändertes Ansprechen auf Substanzen, deren Rezeptoren Gαs aktivieren oder Substanzen, die indirekt Agonisten induzieren, deren Wirkungen über Gαs vermittelt werden. Die geänderte Apoptoseneigung kann eine Vielzahl von Krankheitsverläufen (z. B. von Tumor- und Immunerkrankungen) beeinflussen und das Ansprechen auf Pharmaka bestimmen.

### Verwendung einer Genveränderung in GNAS zur Vorhersage von Erkrankungsrisiken und Krankheitsverläufen

Aufgrund der Schlüsselfunktion der Gas-Untereinheit für die Zellaktivierung ist es ein wesentlicher Bestandteil der Erfindung, dass unter Verweridung von Genveränderungen in GNAS generell Erkrankungsrisiken und Krankheitsverläufe vorhergesagt werden können.

Menschliche heterotrimere G-Proteine sind aus den Untereinheiten α, β und γ zusammengesetzt. Hiervon sind wiederum eine Reihe von Isoformen bekannt, die durch unterschiedliche Gene kodiert werden. Beispielweise gibt es 13 unterschiedliche γ-Isoformen (γ1 - γ13), mindestens 5 unterschiedliche β-Isoformen (β1-β5) und eine Vielzahl unterschiedlicher α-Isoformen (αs (kurz und lang), αo, αi1-3, αq, α11-16, αolf etc.) Da G-Proteine bekannterweise eine zentrale Rolle bei der Steuerung der Funktion aller menschlichen Zellen einnehmen, unabhängig davon, welche Zellrezeptoren aktiviert werden, ist unmittelbar zu erwarten, dass der Verlauf vielfältiger und ganz unterschiedlicher Erkrankungen bei einer genetisch determinierten, verstärkten Aktivierbarkeit von G-Proteinen beeinflusst wird. Gerade bei den vielfältigen Funktionen von G-Proteinen erlangen funktionsverändernde Mutationen eine ganz besonders herausragende Bedeutung und Vorhersagekraft. Diese steht im Gegensatz zu Mutationen in anderen Genen, welche für andere Proteine z.B. Hormone oder Hormonrezeptoren kodieren.

Dies bedeutet, dass durch Genveränderungen in Proteinen, die in allen menschlichen Körperzellen exprimiert werden und dort an zentraler Stelle eingehende Hormonsignale bündeln und dadurch Zellfunktionen regulieren, alle physiologischen und pathophysiologischen Vorgänge entscheidend beeinflusst oder zumindest moduliert werden. Daneben werden auch Antworten auf Pharmaka in besonderer Weise beeinflusst. Hiervon sind erwünschte, aber auch unerwünschte Arzneimittelwirkungen betroffen.

Es wurde in der wissenschaftlichen Literatur wiederholt postuliert, dass Funktionsveränderungen von G-Proteinen einen nachhaltigen Einfluss auf vielfältige Erkrankungen bzw. auf den Verlauf vielfältiger Erkrankungen haben. Solche Genveränderungen können strukturverändernde Mutationen in G-Protein-Untereinheiten sein, die beispielsweise die Aktivierbarkeit durch einen Rezeptor verändern, die enzymatische GTPase Aktivität betreffen oder die Dimerisierung von βγ-Untereinheiten beeinflussen. Daneben könnten solche Veränderungen die Zusammensetzung heterotrimerer G-Proteine verändern. Ferner kann das Expressionsniveau solcher G-Proteinuntereinheiten verändert sein oder es können Spleißvarianten mit geänderter Funktion auftreten (Farfel Z et al., The expanding spectrum of G protein diseases. N Engl J Med. 1999 Apr 1;340(13):1012-20; Iiri T et al., G-protein diseases furnish a model for the turn-on switch. Nature. 1998 Jul 2;394(6688):35-8; Iiri T et al., G proteins propel surprise.Nat Genet. 1998 Jan;18(1):8-10. Spiegel AM. Hormone resistance caused by mutations in G proteins and G protein-coupled receptors. J Pediatr Endocrinol Metab. 1999 Apr;12 Suppl 1:303-9. Spiegel AM. Inborn errors of signal transduction: mutations in G proteins and G protein-coupled receptors as a cause of disease. J Inherit Metab Dis. 1997 Jun;20(2):113-21. Spiegel AM. The molecular basis of disorders caused by defects in G proteins. Horm Res. 1997;47(3):89-96. Spiegel AM. Mutations in G proteins and G protein-coupled receptors in endocrine disease. J Clin Endocrinol Metab. 1996 Jul;81(7):2434-42. Review. Spiegel AM. Defects in G protein-coupled signal transduction in human disease. Annu Rev Physiol. 1996;58:143-70).

Aus den genannten Beispielen geht folgendes hervor:
1. Genveränderungen in Genen, die für ubiquitär exprimierte Proteine kodieren, beeinflussen vielfältige Erkrankungen bzw. verursachen vielfältige Erkrankungsrisiken
2. G-Proteine steuern nahezu alle Signaltransduktionsvorgänge im menschlichen Körper
3. Während aus der zitierten Literatur eindeutig hervorgeht, dass man ganz allgemein davon auszugehen hat, dass Mutationen und Polymorphismen in Genen, die für G-Proteine kodieren, solche Erkrankungen hervorrufen können, so wurde ein Zusammenhang mit genomischen Mutationen im Gen GNAS für die GαS-Untereinheit heterotrimerer G-Proteine mit Erkrankungsrisiken in der Literatur weder beschrieben noch vermutet.

Als Erkrankungen, die mit einer Genveränderung in GNAS einhergehen, und beispielsweise durch ein geändertes Expressionsniveau von Gαs-Protein bestimmt werden, können genannt werden:
1. Herz-Kreislauferkrankungen. Darunter fallen insbesondere Hypertonie, Schlaganfall, koronare Herzkrankheit und Myokardinfarkt, Herzinsuffizienz, Herzrhythmusstörungen, Präeklampsie bzw. Gestose. Da bereits ein Zusammenhang zwischen dem T393C-Polymorphimsmus und Hypertonie beschrieben wurde (Jia H. et al., Association of the Gsα Gene With Essential Hypertension and Response to β-Blockade Hypertension. 1999;34:8-14.), ist es ein Bestandteil der vorliegenden Erfindung, dass die hier neu beschriebenen G(-1211)A-, T2291C- und T(-839)G-Polymorphismen sowie die beschriebenen Polymorphismen im kompletten Kopplungsungleichgewicht (Intron1) ebenfalls ein Risikofaktor für Hypertonie sind, da sie mit dem T393C-Polymorphismus im Kopplungsungleichgewicht stehen. Da Hypertonie wie allgemein bekannt ein Hauptrisikofaktor für Schlaganfall, Herzinfarkt und Herzinsuffizienz ist, ist es ein weiterer Bestandteil der vorliegenden Erfindung, dass die genannten Genveränderungen in GNAS auch das Risiko für solche Erkrankungen erhöhen.
2. Enokrinologische und Stoffwechselerkrankungen. Darunter fallen insbesondere Adipositas, metabolisches Syndrom, Typ-2 Diabetes-mellitus, Gicht, Osteoporose, Schilddrüsenerkrankungen wie Hyper- und Hypothyreose und M.Basedow, Hyper- und Hypoparathyreodismus, Morbus Cushing, Hxper- und Hypoaldosteronismus und viele mehr.
3. Psychiatrische Erkrankungen wie Depression, Schizophrenie, Alkoholismus und Angststörungen, Phobien, Neurosen
4. Neurologische Erkrankungen wie Morbus Parkinson, multiple Sklerose, Epilepsien
5. Dermatologische Erkrankungen wie Psoriasis, Neurodermitis
6. Tumorerkrankungen

### Adipositas

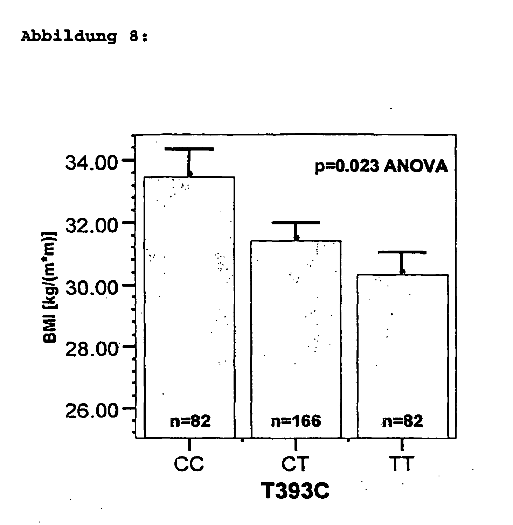

Genetische Studien führten einen genomischen Scan durch, um genetische Marker zu identifizieren, welche mit Adipositas assoziiert sind. Mehrere Arbeiten konnten auf Chromosom 20q13 Adipositas assoziierte Marker identifizieren (Lee, J. H. et al. Genome scan for human obesity and linkage to markers in 20q13. Am:J.Hum.Genet. 64, 196-209 (1999). Dong, C. et al. Interacting genetic loci on chromosomes 20 and 10 influence extreme human obesity. Am.J.Hum.Genet. 72, 115-124 (2003)). Da das GNAS Gen in dieser Region lokalisiert ist, konnte im Rahmen dieser Erfindung gezeigt werden, dass der T393C Polymorphismus signifikant mit dem Körpergewicht assoziiert ist. Hierzu wurden in einer Metaanalyse übergewichtige Patienten mit einem mittleren Körpergewicht von 90.5 kg (BMI = 31.7) Patienten aus 3 unabhängigen klinischen Studien untersucht. Patienten mit CC Genotyp zeigen ein signifikant höheren BMI (33.5 kg/m²) als CT (31.4 kg/m²) und TT Genotypen (30.3 kg/m², Abbildung 8). Abbildung 8 zeigt die Assoziation des GNAS T393C Polymorphismus mit Übergewicht. Gleiche Ergebnisse findet man bei Verwendung der weiteren, hier beschriebnen Polymorphismen.

Adipositas ist ein wesentlicher Risikofaktor für Hypertonie, Schlaganfall, kardiovaskuläre Erkrankungen, Gelenkschäden, Gicht und viele Tumorerkrankungen. Daher ist die Verwendung einer Genveränderung im Gen GNAS zur Vorhersage des Risikos einer Adipositas gleichzeitig dazu geeignet, Risiken für Adipositas-assoziierte Erkrankungen vorherzusagen.

### Genabhängige Serumkonzentration von Leptin und mRNA-Expression im Fettgewebe

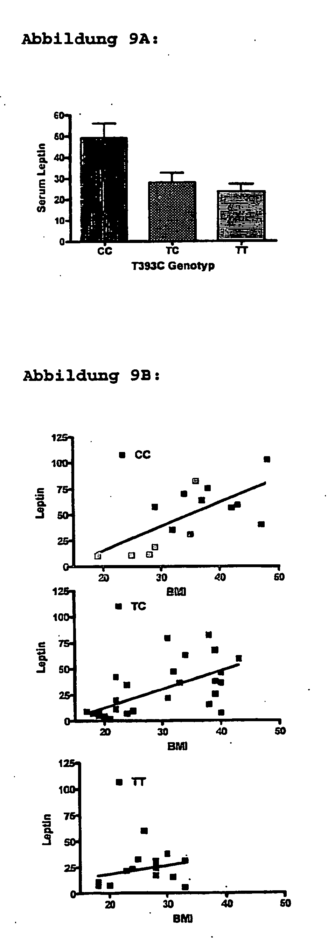

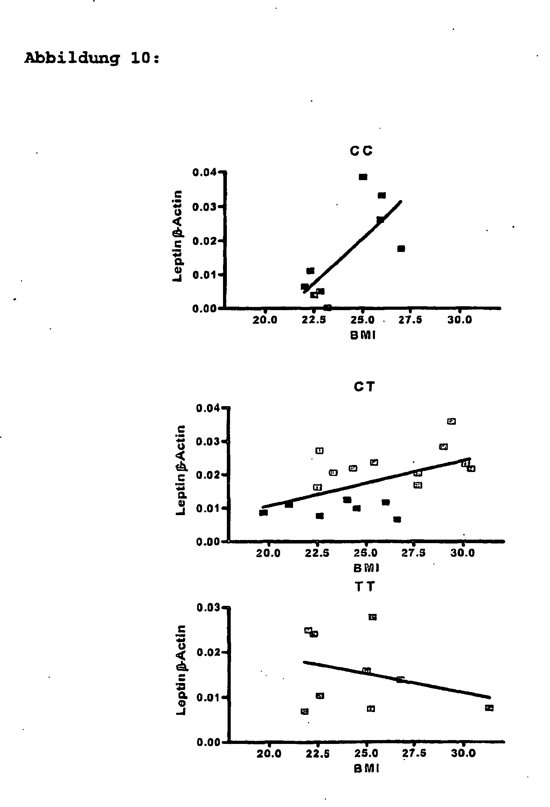

Das Hormon Leptin wird vorwiegend im Fettgewebe produziert und zirkuliert wie alle Hormone im Blutkreislauf des Körpers. Die Wirkung des Leptins wurde durch Experimente bei Mäusen herausgefunden, das heisst, man hat Mäuse gezüchtet ohne das Leptin-Gen (so genannte Knock-out-Mäuse), die einen unstillbaren Appetit zeigen und sehr dick werden. Wenn diesen Mäusen das Leptin injiziert wird, normalisiert sich das Essverhalten und sie werden wieder schlank. Leptin bewirkt somit ein Sättigungsgefühl, eine vermehrte Kalorienabgabe und damit eine gesteigerte Fettverbrennung und Gewichtsabnahme. Wahrscheinlich steuert das Leptin diese Effekte über den Hypothalamus, jenen Teil des Gehirns, wo das Hunger- und Durstzentrum lokalisiert sind. Es hat sich gezeigt, dass Übergewichtige nicht einen Leptinmangel haben, sondern eher eine Resistenz gegen die Leptinwirkung. Daneben gibt es viele Adipöse mit nur geringen Leptin Serumspiegeln. Wir haben untersucht, ob Genveränderungen im Gen GNAS die Serumleptinspiegel bestimmen (Abbildung 9A). Abbildung 9A zeigt die Assoziation des GNAS T393C Polymorphismus mit Serum Leptin. Es zeigt sich bei CC Genotypen eine signifikant (p< 0.005; ANOVA) höhere Leptinkonzentration (49,2) als bei CT (28,0) und TT Genotypen (23,6). Abbildung 9B zeigt die Assoziation des GNAS T393C Polymorphismus mit Serum Leptin in Abhängigkeit vom Body Mass Index (BMI). Es zeigt sich bei CC Genotypen eine signifikant (p< 0.005) größere Steigung nach linearere Regressionsanalyse (2,3; p = 0.0044) als bei CT (1,78; p = 0.0002) und TT Genotypen (0,8; p = 0.3).Bei TT-Genotypen erfolgt kein signifikanter Anstieg des Leptins in Abhängigkeit vom BMI. Zur Bestätigung dieser Befunde wurde die Expression von Leptin-mRNA in humanen Fettgewebe mittels Realtime PCR (Taqman) untersucht (Abbildung 10). Abbildung 10 zeigt die Assoziation des GNAS T393C Polymorphismus mit der Konzentration von Leptin-mRNA im Fettgewebe in Abhängigkeit vom Body Mass Index (BMI). Es zeigt sich bei CC Genotypen eine signifikant (p< 0.005) größere Steigung nach linearer Regressionsanalyse als bei CT und TT Genotypen. Bei TT-Genotypen erfolgt kein signifikanter Anstieg der Leptin-mRNA in Abhängigkeit vom BMI.

Als Kontrolle und für die Normalisierung der Werte diente die Expression von β-Aktin. Das Verfahren ist dem Fachmann bekannt. In Abbildung 10 ist der Quotient Leptin-mRNA/â-Aktin-mRNA als Funktion des BMI dargestellt. Über alle Probanden betrachtet findet man einen linearen Zusammenhang zwischen Leptin-mRNA-Expression und BMI (alle; Steigung 0.0012; p = 0.04). Dieser Zusammenhang ist am Stärksten beim CC-Genotyp (Steigung 0.0049; p = 0.0397), ebenfalls vorhanden beim TC-Genotyp (Steigung 0.0013; p = 0.023), aber fehlend beim TT-Genotyp (Steigung - 0.00008; p = 0,417). Genveränderungen im Gen GNAS bestimmen also die Konzentration von Leptin im Serum und die Expression vom Leptin mRNA in Fettzellen. Eine Genotypisierung bezüglich Genveränderungen im Gen GNAS kann also auch dazu dienen, Patienten zu identifizieren die von einer Therapie mit Leptin oder der Therapie mit Leptinrezeptor - Agonisten profitieren.

### Genabhängige Apoptose von Zellen

Die genabhängige Apotose ist ein streng geregelter physiologischer Vorgang in der Art eines "Zellselbstmords", der für Entwicklung, Erhaltung und Altern vielzelliger Organismen eine wichtige Rolle spielt und bei dem einzelne Zellen planmäßig eliminiert werden. Gemäß der Einbindung der Apoptose in eine große Anzahl von Prozessen, die für die Integrität des Gesamtorganismus verantwortlich sind, gibt es zahlreiche Faktoren, die Apoptose auslösen. Externe Apoptose *auslösende Faktoren* (Signale) sind z. B. Ultraviolett, Röntgenstrahlen, Gammastrahlen, Oxidation, Hitzeschock (heat-shock response), cytotoxische Drogen (Cytotoxine), Schwermetalle. Viele Faktoren (externer oder interner Natur), die oxidativen Stress auslösen, sind gleichzeitig Apoptosesignale, so dass *Sauerstoffradikalen* (freie Radikale) eine entscheidende Bedeutung bei der Apoptoseinduktion zukommt. Bei zahlreichen Krankheiten hat man inzwischen die Beteiligung apoptotischer Prozesse erkannt. Herzerkrankungen (z. B. Herzinfarkt), neurodegenerative Krankheiten (Parkinsonsche Krankheit, AIDS - Untergang der T-Zellen des Immunsystems -, Osteoporose, degenerative Arthritis [Rheumatismus]) sind Beispiele. Andererseits kann Hemmung der Apoptose zu *Krebs* führen. Viele Krebsarten zeichnen sich durch eine Überexpression von Apoptose hemmenden Genen (z. B. *bcl-2*) oder Mutationen in den entsprechenden Genen (z. B. p53; p53-Protein) aus. Einige Substanzen, die in der Tumortherapie eingesetzt werden, induzieren in sensiblen Tumorzellen Apoptose. Zellen des Immunsystems (Immunzellen) unterliegen während ihrer Reifung ebenfalls der Apoptose; T-Lymphocyten, die ein Selbst-Antigen (Autoantigene) erkennen, werden im Thymus (Thymocyten-Entwicklung) durch Apoptose ausgesondert. Dies bedeutet Schutz vor autoreaktiven Zellen (Autoreaktivität). Werden autoreaktive Immunzellen nicht durch Apoptose eliminiert, können daraus Autoimmunkrankheiten resultieren. Wenn genetische Faktoren vorliegen, die generell die Fähigkeit zur Apoptose von Zellen verändern, so ist damit ein generell geändertes Erkrankungsrisiko verbunden und der Verlauf vielfältiger Erkrankungen, bei denen die Apoptose von Bedeutung ist, wird verändert. Dies Betrifft Herz-Kreislauferkrankungen (Myokardinfarkt, koronare Herzkrankheit, Herzinsuffizienz, Schlaganfall), entzündliche Erkrankungen (Rheuma, Gelenkerkrankungen, Morbus Bechterew, Psoriasis, Neurodermitis), Tumorerkrankungen, HIV-Infektion, virale Leberinfektionen (Hepatitis B und C), Abstoßungsreaktionen nach Organtransplantation und vieles mehr.

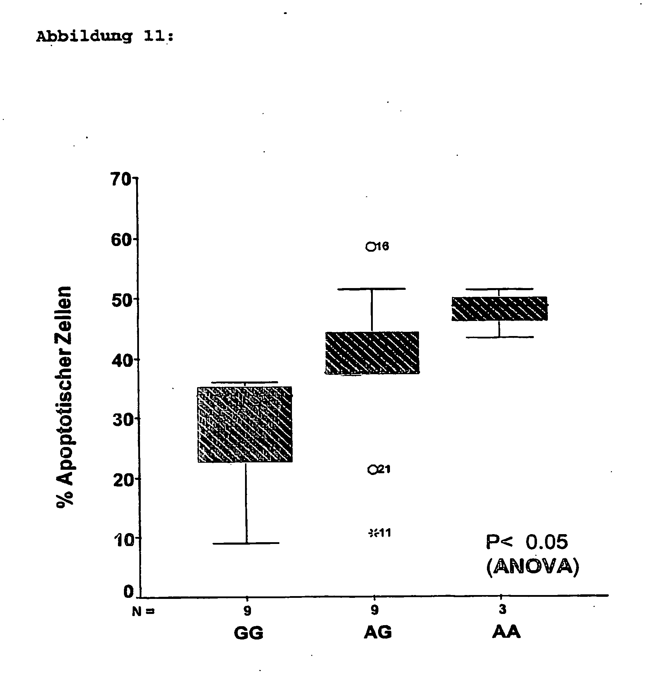

Wir zeigen hier, dass Genveränderungen im Gen GNAS geeignet sind, das spontane Apoptoseverhalten von Zellen zu determinieren. Hierzu wurde die Apoptose bei B-Zellen von Patienten mit chronisch lymphatischer Leukämie untersucht. Die Apoptose wurde nach 24h Inkubation der Zellen in Medium durch FACS-Analyse mittels Annexin V/ PI Färbung quantifiziert. Verfahren zur Quantifizierung der Apoptose sind dem Fachmann bekannt. Die Ergebnisse sind in Abbildung 11 zusammen gefasst.
Abbildung 11 zeigt, dass die Genveränderungen im GNAS-Gen (G(-1211)A- Polymoephismus) die Spontan- Apoptose von B-Zellen bei Patienten mit chronisch lymphatischer Leukämie bestimmen. Die geringste Zahl apoptotischer Zellen wird bei Menschen mit dem GG-Genotyp beobachtet. Die Zellen wurden bezüglich des GNAS G(-1211)A-Polymorphismus genotypisiert. Man erkennt deutlich eine statistisch signifikante verstärkte Apoptose bei Zellen von Individuen mit AA und AG Genotyp und, wie bereits erwähnt, eine relativ dazu verminderte Apoptose bei Zellen von Patienten mit GG-Genotyp. Dieses Verhalten ist übertragbar auf alle anderen Zellen des menschlichen Körpers, die Gás exprimieren.

### Grundlegende Eigenschaften von malignen Tumoren

Bei malignen Tumoren, auch als Krebs bezeichnet, kommt es zu charakteristischen Veränderungen grundlegender Funktionen, die das Wachstum solcher Zellen in ungünstiger Weise befördern. Krebszellen sind gekennzeichnet durch einen Verlust der Kontaktinhibition und ein unkontrolliertes Zellwachstum. Ausgelöst werden solche Veränderungen durch eine Vielzahl von Noxen, sog. Kanzerogene, welche das Erbgut schädigen. Zu solchen Noxen gehören viele Chemikalien, Tabakrauch, aber auch UV-Licht. Daneben spielen genetische Faktoren bei der Krebsentstehung eine herausragende Rolle. Kennzeichnend für Krebszellen ist neben ihrem ungehemmten Wachstum auch die Neigung, Tochtergeschwülste (Metastasen) in anderen Organen abzusiedeln. Die Ausbreitung der Metastasen erfolgt regelmäßig über die Blutbahn oder über Lymphgefäße. Krebserkrankungen sind bei einem Großteil der Fälle nicht heilbar und führen zum Tode. Therapeutisch wird versucht, den Ausgangstumor und Metastasen operativ zu entfernen. Daneben können Tumore bestrahlt werden. Mittels sogenannter Zytostatika, Antikörper gegen bestimmte Proteine oder Oberflächenmarker oder immunmodulierender Substanzen (Zytokine, Interferone) wird versucht, die sich schnell teilenden Krebszellen abzutöten oder in den programmierten Zelltod (Apoptose)zu überfahren. Die derzeit verfügbaren therapeutischen Maßnahmen führen in den meisten Fällen nur zu einem verlängerten Überleben, nicht zur definitiven Heilung.

### Prognosefaktoren bei Krebserkrankungen

Es ist von medizinisch überaus hoher Relevanz, Prognosefaktoren für den Verlauf von Krebserkrankungen zu definieren, die Auskunft über das Ansprechen auf bestimmte Therapieformen geben oder die generell prädiktiv für das Auftreten von Metastasen, die Tumorprogression und das Überleben sind. Bislang werden in der Medizin dem Fachmann allgemein bekannte Prognosefaktoren eingesetzt. Hierzu gehören beispielsweise die Größe des Tumors, seine Eindringtiefe in das umgebende Gewebe, organüberschreitendes Wachstum, das Eindringen in Blut- oder Lymphgefäße oder in Lymphknoten, sowie der Differenzierungsgrad der Tumorzellen. Daneben existieren einige relativ unspezifische serologische Marker. Das Verfahren zur Klassifikation der Tumore wird generell als "Staging" und "Grading" bezeichnet. Generell gilt, dass das Vorhandensein von Fernmetastasen und ein geringer Differenzierungsgrad prognostisch sehr ungünstige Parameter darstellen. Dennoch ist es die allgemeine Erfahrung in der Medizin, dass Patienten bei gleichem Tumorstadium drastisch unterschiedliche Krankheitsverläufe aufweisen. Während man bei manchen Patienten eine schnelle Progression der Erkrankung und das Auftreten von Metastasen und Rezidiven beobachtet, kommt die Erkrankung bei anderen Patienten aus unklaren Gründen zum Stillstand. Metastasen können hierbei lokal, regional und fern der Muttergeschwulst auftreten. Dazu ist es notwendig, dass eine hohe Zahl von Geschwulstzellen über den Lymph- oder Blutweg durch einfache Fortschwemmung in Flüssigkeit oder durch Abklatschen in benachbartes Gewebe gelangt. Unter Rezidivneigung versteht man das erneute Auftreten einer Geschwulst nach operativer unvollständiger oder Teilentfernung des Tumors. Hierbei handelt es sich nicht um eine erneute maligne Transformation, sondern um das Nachwachsen eines nicht vollständig entfernten Tumorgewebes. Eine Rezidivbildung ist auch durch Metastasen möglich, die viele Jahre latent bleiben können. Unter Progression versteht man das Wiederauftreten eines Tumors mit höherem Grading (mehr Entdifferenzierung) oder das Neuauftreten von Metastasen.

Ganz offensichtlich gibt es eine Vielzahl individueller, unerkannter biologischer Variablen die den Verlauf einer Tumorerkrankung unabhängig von Staging und Grading in hohem Masse determinieren. Zu solchen Faktoren gehören genetische Wirtsfaktoren.

Daneben ist es wünschenswert, genetische Marker zu entwickeln, die prädiktiv für das Auftreten von Tumoren sind. Solche Marker erfüllen die Funktion, die betroffenen Individuen frühzeitig weiteren Screeningmassnahmen (Serologie, Röntgen, Ultraschall, NMR etc.) zuzuführen. Damit können Krebserkrankungen im Frühstadium erkannt und therapeutisch angegangen werden, wobei die Heilungs- und Überlebenschancen bei Tumoren im Frühstadium wesentlich besser sind als bei fortgeschrittenen Tumoren.

### Arten von Tumoren

Generell können alle Zellen des menschlichen Körpers maligne entarten und zu einer Krebserkrankung führen. Die hier und im weiteren gemachten Ausführungen beschreiben generelle Mechanismen der Tumorprogression, der Metastasierung und des therapeutischen Ansprechens. Insofern gelten die hier beschriebenen Mechanismen und Ansprüche für alle Tumore des Menschen, beispielsweise für die folgende Tumoren:
Tumoren des Urogenitaltrakts: Hier sind zu nennen das Harnblasenkarzinom, das Nierenzellkarzinom, das Prostatakarzinom und das Seminom.
Tumoren der weiblichen Geschlechtsorgane: Das Mammakarzinom, das Korpuskarzinom, das Ovarialkarzinom, das Zervixkarzinom.
Tumoren des Gastrointestinaltraktes: Das Mundhöhlenkarzinom,
das Ösophaguskarzinom, das Magenkarzinom, das Leberkarzinom, das Gallengangskarzinom, das Pankreaskarzinom, das Kolonkarzinom, das Rektumkarzinom.
Tumore des Respirationstraktes: das Kehlkopfkarzinom, das Bronchialkarzinom.
Tumore der Haut: das maligne Melanom, das Basaliom, das T-zell-Lymphom
Tumorerkrankungen des blutbildenden Systems: Hodgkin- und non-Hodgkin- Lymphome, akute und chronische Leukämien etc.
Tumorerkrankungen des Gehirns bzw. des Nervengewebes: Glioblastom, Neuroblastom, Medulloblastom, meningeales sarkom, Astrozytom.
Weichteiltumore, beispielsweise Sarkome und Kopf-Hals-Tumore.

### G Proteine und maligne Transformation von Zellen

Experimentelle in vitro Untersuchungen belegen, dass Mutationen in G-Proteinuntereinheiten eine maligne Transformation von Zellen bewirken können. Die Expression einer konstitutiv aktiven Gαq-Untereinheit mit fehlender GTPase-Aktivität führt zur malignen Transformation von Fibroblasten (Kalinec G et al., Mutated alpha subunit of the Gq protein induces malignant transformation in NIH 3T3 cells. Mol Cell Biol. 1992 Oct;12(10):4687-93). Ähnliche Beobachtungen wurden für die α-Untereinheiten Gα12, Gα13, Go, Gz und Gαi2 gemacht (Xu N et al., A mutant alpha subunit of G12 potentiates the eicosanoid pathway and is highly oncogenic in NIH 3T3 cells. Proc Natl Acad Sci U S A. 1993 Jul 15;90(14):6741-5. Xu N et al Potent transforming activity of the G13 alpha subunit defines a novel family of oncogenes. Biochem Biophys Res Commun. 1994 Jun 15;201(2):603-9. Pace AM et al A mutant alpha subunit of Gi2 induces neoplastic transformation of Rat-1 cells. Proc Natl Acad Sci U S A. 1991 Aug 15;88(16):7031-5. Wong YH et al., Mutant alpha subunit of Gz transforms Swiss 3T3 cells. Oncogene. 1995 May 18;10(10):1927-33). Diese Arbeiten belegen, dass Mutationen in α-Untereinheiten prinzipiell zur malignen Zelltransformation beitragen können.

### G-Protein-Mutationen und Krebserkrankungen beim Menschen

Beim Menschen wurden bei einigen selten Adenomen somatische Mutationen in den Untereinheiten Gαs und Gα12 nachgewiesen. Diese werden als Gip2 (Gai2-Untereinheit)bzw. als Gsp (Gas-Untereinheit) bezeichnet. Hierbei handelt es sich dagegen nicht um genetische Wirtsfaktoren, die den Krankheitsverlauf modulieren, sondern um kausal wirkende Faktoren (Übersicht bei: Farfel Z et al. The expanding spectrum of G protein diseases. N Engl J Med. 1999;340(13) :1012-20).

### Verwendung von Genveränderungen im Gen GNAS zur Vorhersage des Verlaufs von Tumorerkrankungen

Ein wesentlicher Bestandteil der vorliegenden Erfindung ist die Bereitstellung diagnostisch relevanter Genveränderungen im Gen GNAS als Prognosefaktor für alle Tumorerkrankungen des Menschen. Naturgemäß können hierbei nicht alle Tumorerkrankungen beschrieben werden. Das Prinzip wird daher an ausgewählten Beispielen erläutert, welche die generelle Verwendbarkeit demonstrieren:

### Beispiel 1: Chronisch lymphatische Leukämie (CLL)

Bei der chronischen lymphatischen Leukämie handelt es sich um eine chronisch verlaufende Form einer Leukämie. Charakteristisch für die Krankheit ist eine große Zahl von entarteten Lymphozyten. Insgesamt 30 Prozent aller leukämischen Erkrankungen sind chronisch lymphatische Leukämien. Das mittlere Erkrankungsalter liegt bei 65 Jahren. Nur zehn Prozent der Patienten sind jünger als 50 Jahre. Männer sind etwa zwei- bis dreimal häufiger betroffen als Frauen. Risikofaktoren für die Entstehung einer CLL sind nicht bekannt. Jedoch tritt die Krankheit in Japan und China selten auf. Auch Japaner, die in die USA eingewandert sind, erkranken äußerst selten an einer CLL. Diese Tatsache deutet darauf hin, dass genetische Faktoren eine Rolle spielen. Die Therapie richtet sich nach dem Stadium der Erkrankung. Eine CLL kann bis zu 20 Jahre lang gutartig verlaufen, das heißt, der Patient zeigt außer vergrößerten Lymphknoten und eventueller Müdigkeit sowie Appetitlosigkeit keine Symptome. Die Behandlung setzt erst dann ein, wenn die Zahl der Lymphozyten über ein bestimmtes Maß anzusteigen beginnt, der Anteil der roten Blutkörperchen und die Zahl der Blutplättchen absinkt oder andere Komplikationen auftreten. Eine frühzeitige Behandlung hat keinen Einfluss auf den Verlauf und den Ausgang der Krankheit. Die wichtigste therapeutische Maßnahme ist die Chemotherapie. In bestimmten Fällen muss sich der Patient auch bestrahlen oder operieren lassen. Patienten können bis zu 20 Jahre mit der Diagnose CLL leben, ohne dass sich schwer wiegende Symptome zeigen. Je weiter die Erkrankung fortgeschritten ist, desto größer sind allerdings auch die Gesundheitsstörungen durch die Veränderung des Organsystems. Je nach Binet-Stadium der Krankheit kann der Arzt die Prognose abschätzen. Das Stadium einer.CLL ist unter anderem dadurch gekennzeichnet, wie viele Lymphozyten sich im Blut und Knochenmark befinden, wie groß Milz und Leber sind und ob eine Blutarmut vorliegt oder nicht. Eine CLL führt zu Veränderungen im Immunsystem, so dass Menschen, die an dieser Krankheit leiden, stärker gefährdet sind, andere Arten von Krebs zu entwickeln. Bei gleichem Bindet-Stadium zeigen Patienten jedoch ganz unterschiedliche Krankheitsverläufe. Bestandteil der Erfindung ist es zu zeigen, dass Genveränderungen im Gen GNAS geeignet sind, den Verlauf der CLL vorherzusagen. Hierzu wurden Patienten mit CLL bezüglich der beschrieben Genveränderungen in GNAS genotypisiert und der Genstatus mit der Krankheitsprogression verglichen. Unter Progression definieren wir hier das Zeitintervall zwischen Erstdiagnose der CLL und der Therapiebedürftigkeit.

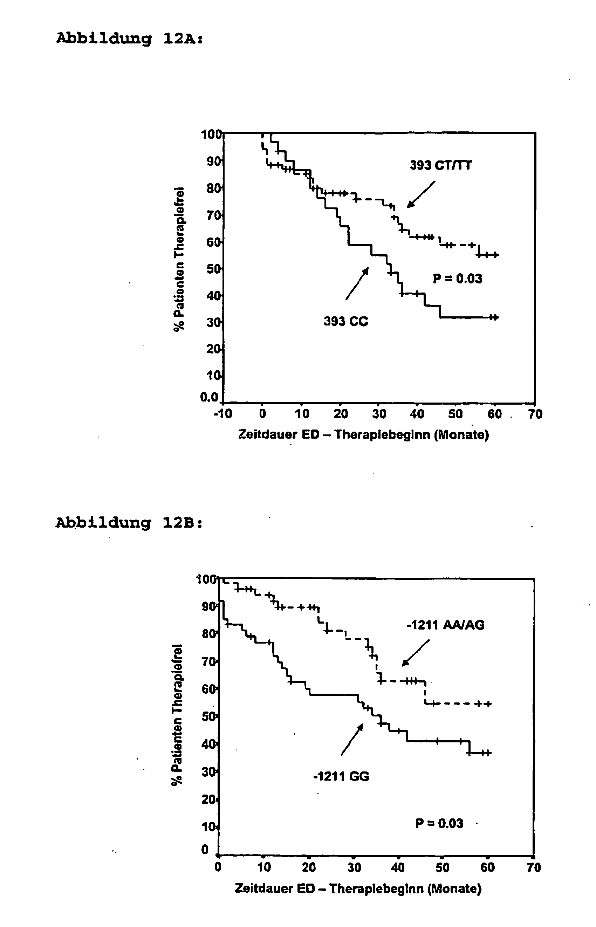

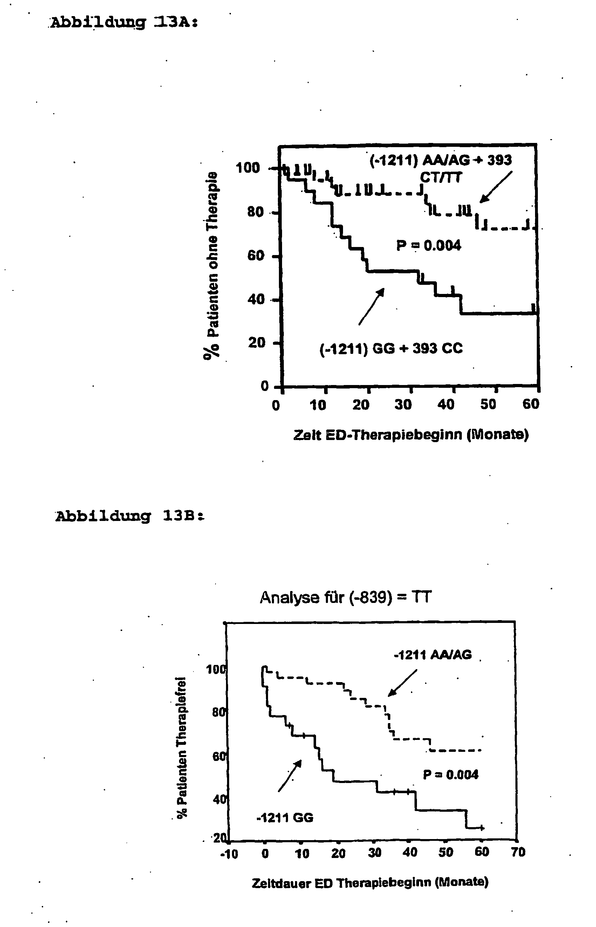

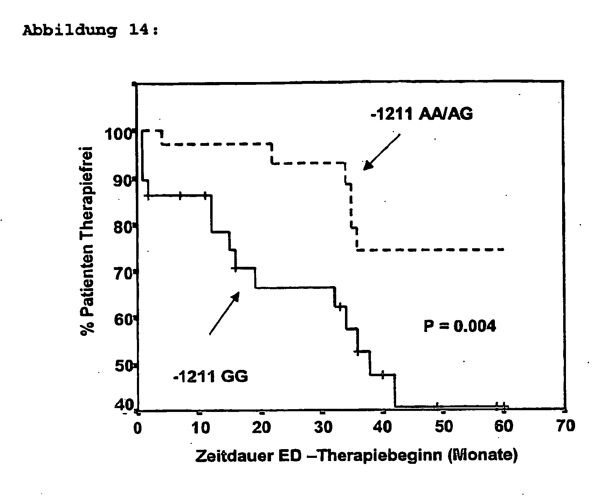

Abbildung 12A zeigt den GNAS T393C-Status und die Krankheitsprogression bei Patienten mit CLL (ED = Erstdiagnose). Es wird deutlich, dass ein signifikanter Unterschied bezüglich Therapiefreiheit in Abhängigkeit vom GNAS T393C-Status vorliegt, wobei der CC-Genotyp mit einer um das Zweifache beschleunigten Krankheitsprogression assoziiert ist (Hazard Ratio 2.07; p = 0.02). Abbildung 12B zeigt den GNAS G(-1211)A-Status und die Krankheitsprogression bei Patienten mit CLL (ED = Erstdiagnose). Hier wird deutlich, dass ein signifikanter Unterschied bezüglich Therapiefreiheit in Abhängigkeit vom GNAS G(-1211)A-Status vorliegt, wobei der GG-Genotyp mit einer um das Dreifache beschleunigten Krankheitsprogression assoziiert ist (Hazard Ratio 3.01; p = 0.01). Besonders stark wird der Effekt bei kombinierter Analyse der Allele bzw. Genotypen (-1211) GG + 393 CC versus (-1211)AG/AA + 393 CT/TT (Abbildung 13A). In ähnlicher Weise können Analysen des G(-1211)A-Status und des T(-839)G-Status kombiniert werden, wie dies in Abbildung 13B gezeigt wird. Auch die Kombination mit dem T2291C Status ist sinnvoll, da auch dieser Polymorphismus sowohl mit dem G(-1211)A- als auch mit dem T393C Polymorphismus im Kopplungsungleichgewicht liegt. Abbildung 14 belegt, dass Genveränderungen in GNAS zur Prognose der CLL besonders auch im Frühstadium (BINET A) herangezogen werden können, wie das für den G(-1211)A Status gezeigt wird.

### Beispiel 2 : Harnblasenkarzinom

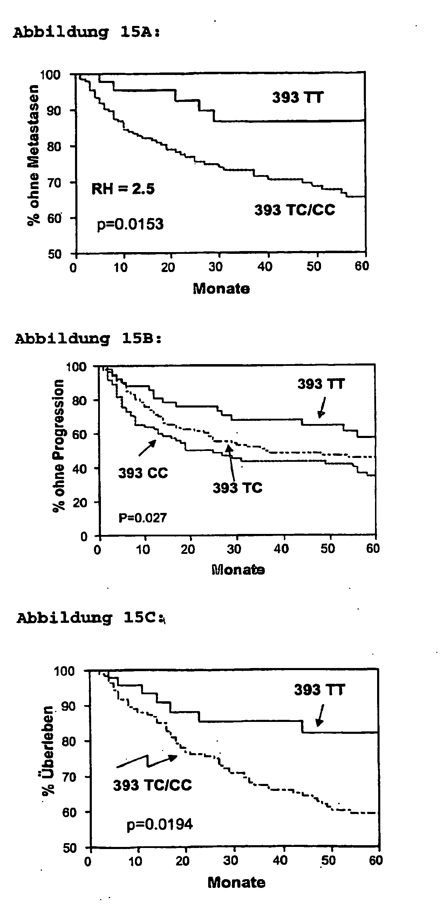

Blasenkrebs ist ein bösartiger Tumor an der Schleimhaut der Blase. Blasenkrebs tritt am häufigsten zwischen dem 60. und 70. Lebensjahr auf. Männer sind davon dreimal so häufig betroffen wie Frauen. Bei Männern ist Blasenkrebs nach Lungen- und Prostatakrebs die dritthäufigste Krebsform. Blasenkrebs kann durch äußere Einflüsse hervorgerufen werden. Zu den Risikofaktoren gehören, Rauchen, ständige Belastung des Organismus durch Chemikalien beispielsweise Farbstoffe, Schmerzmittelmissbrauch. Bei vielen Patienten ergeben die Untersuchungen, dass es sich um einen oberflächlichen Tumor handelt. Dieser kann operativ mit Hilfe des Zystoskops entfernt werden. Mehr als 70 % der Patienten, die wegen eines oberflächlichen Blasenkarzinoms behandelt wurden, zeigen im Verlauf ein Tumorrezidiv. Dabei kommen in mehr als der Hälfte Rezidivtumoren mit nichtmuskelinvasiver Erkrankung vor. Diese können durch transurethrale Resektion kurativ behandelt bzw. kontrolliert werden. Es ist deshalb wichtig, diese Läsionen frühzeitig zu erkennen und den Patienten regelmäßig und engmaschig nachzusorgen. Dabei steht an 1. Stelle die Zystoskopie mit Urinzytologie. In regelmäßigen Intervallen dienen Ausscheidungsurogramme zur Kontrolle möglicher Tumormanifestationen in Nierenbecken und Harnleitern. Bislang gibt es kaum valide Marker, die für den weiteren Verlauf der Erkrankung prädiktiv sind. Derzeit werden daher die klassischen Faktoren wie Eindringtiefe, Differenzierungsgrad, Metastasierung, Lymphknotenbefall etc. für die Prognose herangezogen. Genetische Marker für Tumorprogression, Rezidivneigung, Uberlebenswahrscheinlichkeit und Therapieansprechen würden die Betreuung von Patienten mit Harnblasenkarzinom wesentlich verbessern. Ein weiterer Bestandteil der Erfindung besteht darin, dass die Verwendung von Genveränderungen in GNAS dazu geeignet ist den weiteren verlauf der Erkrankung vorherzusagen. Abbildung 15A zeigt den Zeitpunkt bis zum Auftreten von Metastasen in Abhängigkeit vom GNAS T393C- Status. Hierbei ist das Metastasierungsririko bei Patienten mit TC/CC Genotyp um das 2,5-fache Erhöht (95 % : 1,2- 4.0; p = 0.015). Die mediane Zeit bis zur Metastasierung beträgt 29 Monate bei TT-Genotyp, hingegen 13 Monate beim kombinierten TC/CC-Genotyp. Eine ähnliche Beziehung wird gefunden, wenn die Zeit bis zur Tumorprogression untersucht wird (Abbildung 15B). Hierunter verstehen wir, das Auftreten von Metastasen oder das Wiederauftreten des Tumors mit höherem Staging oder Grading. Der Kurvenverlauf ist für die 393 TT-, TC- und CC- Genotypen signifikant verschieden (p = 0.027, Logrank-Test, wobei den CC- und TC-Genotypen der ungünstigere Verlauf zugeordnet wird. Schließlich wird der Zusammenhang zwischen GNAS T393C-Status und Überleben dargestellt (Abbildung 15C). Auch hier erkannt man wiederum, dass Patienten mit den GNAS 393-Genotypen TC und CC früher versterben als Patienten mit dem TT-Genotyp. Die Überlebenszeit beträgt im Median 44 Monate bei Patienten mit dem TT-Genotyp, hingegen nur 20,5 Monate für den kombinierten TC/CC-Genotyp. Wegen des signifikanten Kopplungsungleichgewichts zum T393C-Polymorphismus kann in ähnlicher Weise der G(-1211)A- und der T2291C- Polymorphismus verwendet werden. Es ist davon auszugehen, dass eine verstärkte Apoptoseneigung bedingt durch eine vermehrte Gαs -Expression bei T393-Trägern bzw. -1211A- oder 2291C Trägern den Erkrankungsverlauf günstig beeinflusst.

### Beispiel 3: Nierenzellkarzinom

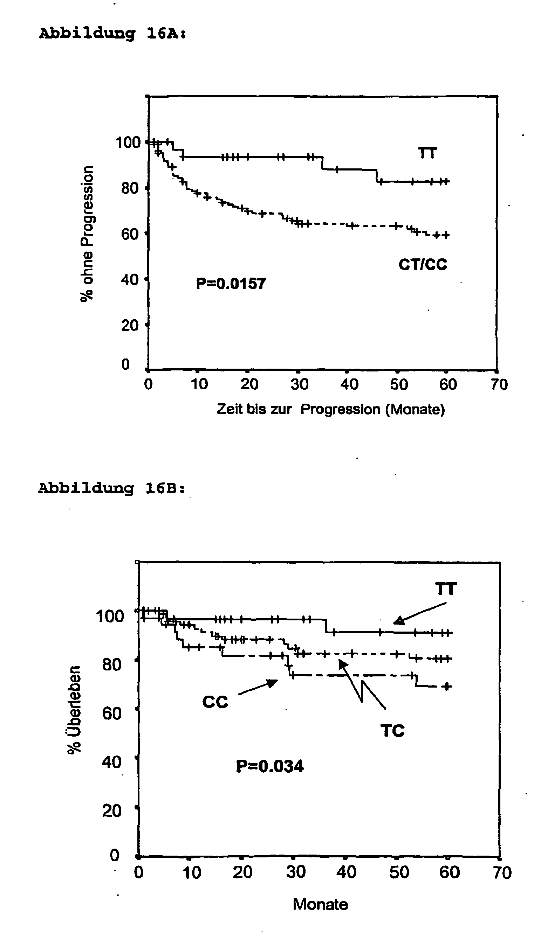

Nierenkrebs ist eine schwer wiegende Krankheit. Die Heilungschancen sind abhängig von der Tumorgröße und von der Tumorausbreitung. Bei Patienten ohne Metastasen beträgt die 10-Jahres-Überlebensrate bis zu 80%, allerdings mit einer erheblichen interindividuellen Variabilität. Durch den heute weit verbreiteten Einsatz von Ultraschalltechnik werden viele der Tumoren bereits in einem frühen nicht metastasierten Stadium erkannt und können so rechtzeitig behandelt werden. Bei einem Vorliegen von Fernmetastasen ist es möglich, die operative Entfernung der Niere mit einer anschließenden Immuntherapie mit Interleukin-2 oder Interferon-Alpha zu kombinieren. Dadurch wird die körpereigene Krebszellabwehr gestärkt. Beim metastasierten Nierenzellkarzinom kann mit der Kombination von Interferon, Interleukin-2 und 5-Fluorouracil in Studien eine Ansprechrate von 36 Prozent erreicht werden. Derzeit gibt es keine prädiktiven Marker für die Progression, das Überleben oder Therapieansprechen bei Patienten mit Nierenzellkarzinom. Unter Verwendung des GNAS T393C-Polymorphismus erkannte man eine beschleunigte Progression bei Patienten mit den TC/CC-Genotypen (Abbildung 16 A). Ferner wird hier gezeigt, dass das Überleben bei Patienten mit Nierenzellkarzinom vom GNAS-T393C-Status abhängt (Abbildung 16B). Hier versterben die Patienten mit dem CC-Genotyp am frühesten, gefolgt von den TC- und TT-Genotypen, wobei letztere am längsten überleben. In der multivariaten Analyse sind GNAS-Status (p= 0.012); Grading (p < 0.001) und Stadium (p < 0.001) unabhängig Prädiktoren für das Überleben. Wegen des signifikanten Kopplungsungleichgewichts zum T393C-Polymorphismus kann in ähnlicher Weise der G(-1211)A-Polymorphismus verwendet werden.

### Beispiel 4: Akute myeloische Leukämie (AML)

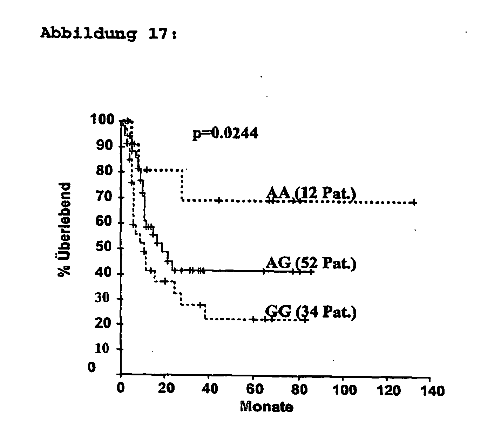

Die Zellen mit der größten Bedeutung für die AML sind die Granulozyten und die Monozyten. Die Hauptfunktion dieser Zellen ist die Immunabwehr. Der AML liegt eine Entartung der Stammzelle zugrunde. Diese Entartung führt dazu, dass die Stammzelle die Fähigkeit zur Differenzierung in funktionstüchtige Blutzellen verliert. Voll erhalten bleibt dabei ihre Vermehrungstendenz. Es kommt so zu einer bösartigen Vermehrung von unreifen Zellen, den Blasten. Diese wuchern im Knochenmark zu schnell, zu unkontrolliert und werden zu früh in das Blut entlassen. Somit erlangen sie nicht die erforderliche Reife und können deshalb auch nicht ihren Aufgaben wie zum Beispiel der Infektioüsabwehr nachkommen. Da der Raum im Knochenmark begrenzt ist, verdrängen die Leukämiezellen die heranwachsenden roten Blutkörperchen und Blutplättchen. Es werden zuwenig Erythrozyten und Thrombozyten produziert. Folgen sind eine Blutarmut und eine übermäßige Blutungsneigung. Da die Leukämiezellen durch das Blut- und Lymphgefäßsystem zirkulieren, wandern sie auch in Lymphknoten, Milz und Leber und führen dort zu einer Funktionsbeeinträchtigung. Oft kommt es dadurch zu einer Größenzunahme dieser Organe. Die akute myeloische Leukämie ist also als bösartige Expansion und Verlust der Differenzierungsfähigkeit blutbildender Zellen im Knochenmark aufzufassen. Betroffen sind dabei vor allem die Zellen der myeloischen Reihe, also Monozyten und Granulozyten. Grundlegender Bestandteil der Behandlung von Patienten mit akuter myeloischer Leukämie ist die Chemotherapie. Dabei wird eine Wachstumshemmung der Leukämiezellen durch die Gabe von Medikamenten versucht. Solche Medikamente werden auch als Zytostatika bezeichnet. Ergänzt werden kann die Chemotherapie durch zwei weitere Therapieoptionen: die Stammzelltransplantation und die Knochenmarkstransplantation.
Leider ist es bisher nicht gelungen, Medikamente zu entwickeln, die selektiv die Leukämiezellen schädigen. Die Nebenwirkungen der Chemotherapie haben ihre Ursache in einer Schädigung gesunder, sich natürlicherweise rasch teilender Zellen. Zytostatika, also Medikamente, welche die Zellteilung stören, werden meist in Kombinationen und nach einem zeitlich festgelegten Schema verabreicht. In ca. 70 bis 80 % wird nach 2 bis 3 Behandlungszyklen eine vollständige Remission, also eine Rückbildung aller Krankheitszeichen erreicht. Durch intensive Chemotherapie werden 99 bis 99,9 % aller Leukämiezellen zerstört. Die verbleibenden leukämischen Zellen lassen sich aber bei der Mehrzahl der Patienten trotz intensiver Konsolidierungstherapie nicht vernichten. Nach 5 Jahren leben daher nur noch 20 bis 40 % der Patienten. Es wurde nun untersucht, ob Genveränderungen im GEN GNAS als Prognosefaktoren für das Überleben bei Patienten mit AML verwendet werden können. Es wurde ein signifikante Zusammenhang mit dem Genotypstatus bezogen auf den A(-1211)G- Polymorphismus gefunden. Abbildung 17 zeigt, dass Patienten mit akuter myeloischer Leukämie mit dem AA-Genotyp des A(-1211)G- Polymorphismus die beste Überlebenschance haben, während der Krankheitsverlauf bei AG- und GG-Genotypen beschleunigt ist. Alle in die Studie eingeschlossenen Patienten hatten eine intensive Chemotherapie und/oder eine Knochenmarktransplantation erhalten. Damit wird nicht nur gezeigt, dass Genveränderungen im Gen GNAS Marker für die Progression der AML sind, sondern dass diese Genveränderungen auch als allgemeine pharmakogenetische Marker für Krebschernotherapeutika und Knochenmarktransplantation eingesetzt werden können.

### Mögliche molekulare Ursachen der GNAS-abhängigen Tumorprogression

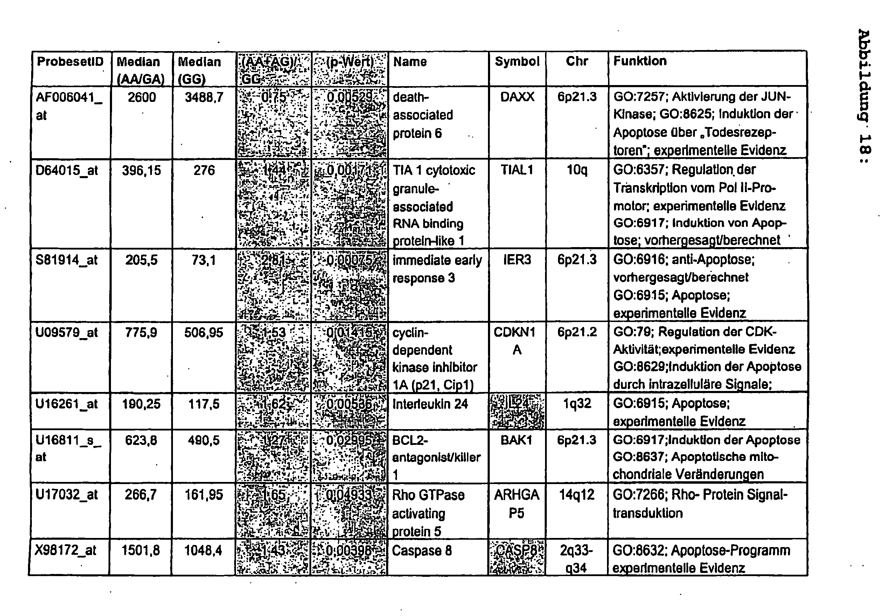

Hierzu wurde cDNA von CLL-Zellen mit Affymetrix-Genchips hybridisiert wie in der Literatur beschrieben (Dürig J et al., Expression of ribosomal and translation-associated genes is correlated with a favorable clinical course in chronic lymphocytic leukemia. Blood. 2003 Apr 1;101(7):2748-55). Das Expressionsniveau der Gene wurde nicht nach dem klinischen Verlauf, sondern nach dem Status des GNAS G(-1211)A-Polymorphismus untersucht. Das Ergebnis ist in Abbildung 18 gezeigt. Abbildung 18 zeigt die Genexpression bei Lymphozyten von Patienten mit CLL in Abhängigkeit vom GNAS G(-1211)A-Genotyp. Die CDNA wurde, wie bereits erwähnt, auf Genchips von Affymetrix hybridisiert und die Stärke des Hybridisierungssignals quantifiziert. Das Verfahren zur quantitativen Genanalyse ist dem Fachmann bekannt. Man erkennt eine statistisch signifikante, stärkere Expression Apoptose-relevanter Gene bei AA/AG-Genotypen gegenüber dem GG-Genotyp. Natürlich kann hier nicht eine Darstellung aller der auf dem Affymetrix-Chip befindlichen 30.000 Gene erfolgen. Interessanterweise findet man bei (-1211)A-Allelträgern im Vergleich zum GG Genotyp eine signifikante Mehrexpression Apoptose-assoziierter Gene, z.B. von Caspase 8, immediate early response 3, IL24, CDKN1a usw. Damit ist die Vermutung naheliegend, dass eine verstärkte Signaltransduktion über Gαs auf Grund der vermehrten Expression zu einer verstärkten Apoptoseneigung beiträgt. Dies zeigt auch, dass Genveränderungen im GNAS-Gen dazu verwendet werden können, krankheitsrelevante Gene und neue Angriffspunkte für Pharmaka zu entdecken.

### Verwendung von Genveränderungen im Gen GNAS zur Vorhersage von Krankheitsrisiken und Verläufen

Da die vielfältigen Funktionen von Gαs gut bekannt sind, können Genveränderungen im Gen GNAS das Risiko für viele unterschiedliche Krankheiten erhöhen bzw. Krankheitsverläufe beeinflussen. Es ist generell nicht möglich, alle Krankheiten des Menschen und der Verläufe zu untersuchen. Wir haben dies hier jedoch exemplarisch für vier unterschiedliche Krankheiten gezeigt: die Adipositas, CLL, AML, Harnblasenkarzinom und Nierenzellkarzinom. Diese Daten belegen eindeutig die Verwendbarkeit der Genveränderungen im Gen GNAS für den hier beschriebenen Zweck. Diese Erkrankungen stehen a priori in keinerlei Zusammenhang.

### Pharmakogenetik - Diagnostik der Wirksamkeit von Pharmaka, der Potenz und Effizienz von Pharmaka und dem Auftreten unerwünschter Wirkungen

### Grundlagen und Ziele der Pharmakogenetik

Die Wirksamkeit von Arzneimitteln und/oder das Auftreten unerwünschter Nebenwirkungen wird neben den spezifischen Stoffeigenschaften der chemisch definierten Produkte durch eine Reihe von Parametern definiert. Zwei wichtige Parameter, die erzielbare Plasmakonzentration und die Plasmahalbwertszeit bestimmen in hohen Maße die Wirksamkeit oder Unwirksamkeit von Pharmaka oder das Auftreten unerwünschter Wirkungen. Die Plasmahalbwertszeit wird unter anderem dadurch bestimmt mit welcher Geschwindigkeit bestimmte Pharmaka in der Leber oder anderen Körperorganen zu wirksamen oder unwirksamen Metaboliten verstoffwechselt und mit welcher Geschwindigkeit sie aus dem Körper ausgeschieden werden, wobei die Ausscheidung über die Nieren, über die Atemluft, über den Schweiß, über die Spermaflüssigkeit, über den Stuhl oder über andere Körpersekrete erfolgen kann. Daneben wird die Wirksamkeit bei oraler Gabe durch den sog. "first-pass-Effekt" limitiert, da nach Resorption von Pharmaka über den Darm ein bestimmter Anteil in der Leber zu unwirksamen Metaboliten verstoffwechselt wird.

Mutationen oder Polymorphismen in Genen metabolisierender Enzyme können die Aktivität derselben in der Weise verändern, dass deren Aminosäurezusammensetzung verändert wird, wodurch die Affinität zum metabolisierende Substrat erhöht oder erniedrigt wird und damit der Metabolismus beschleunigt oder verlangsamt sein kann. In ähnlicher Weise können Mutationen oder Polymorphismen in Transportproteinen die Aminosäurezusammensetzung in der Weise verändern, dass der Transport und damit die Ausscheidung aus dem Körper beschleunigt oder verlangsamt wird.

Zur Auswahl der für einen Patienten optimal geeigneten Substanz, der optimalen Dosierung, der optimalen Darreichungsform und zur Vermeidung unerwünschter, z.T. gesundheitsschädlicher oder tödlicher Nebenwirkungen ist die Kenntnis von genetischen Polymorphismen oder von Mutation, die zur Änderung der Genprodukte führen, von herausragender Bedeutung.

### Die Wirkung von Hormonen im menschlichen Körper und die Bedeutung von Polymorphiämen in Hormonreaeptorea

Ein Vielzahl von Hormonen und Peptidhormonen des menschlichen Körpers aber auch Rezeptorantagonisten üben ihre Wirkung an sogenannten Rezeptoren der Körperzellen aus. Dies sind Proteine unterschiedlicher Zusammensetzung. Nach Aktivierung dieser Rezeptoren müssen diese Signale ins Zellinnere geleitet werden, was über die Aktivierung von heterotrimeren G-Proteinen vermittelt wird. Solche G-Proteine sind aus unterschiedlichen α-, β- und γ- Untereinheiten zusammengesetzt. Diese Rezeptoren lassen sich, je nach Aktivierbarkeit durch definierte Hormone, in bestimmte Gruppen unterteilen. Dem Fachmann ist bekannt, dass Mutationen oder Polymorphismen in bestimmten Rezeptoren die Wirksamkeit bestimmter Agonisten oder Antagonisten an diesen Rezeptoren determinieren können. So beeinflusst ein häufiger Gly16Arg- Polymorphismus im Gen, das für den β2-Adrenozeptor kodiert, die Stärke der Ansprechbarkeit auf das β2-Sympathomimetikum Salbutamol (Martinez FD, et al. Association between genetic polymorphisms of the beta2-adrenoceptor and response to albuterol in children with and without a history of wheezing. J Clin Invest. 1997 Dec 15;100(12):3184-8). Polymorphismen im D2-Rezeptorgen bestimmen die Häufigkeit des Auftretens von Dyskinesien bei der Behandlung des Morbus Parkinson (Parkinson's disease) mit Levadopa (Oliveri RL, et al.; Dopamine D2 receptor gene polymorphism and the risk of levodopa-induced dyskinesias in PD. Neurology. 1999 Oct 22;53(7):1425-30): Polymorphismen im µ-Opiatrezeptor-Gen bestimmen die analgetische Wirksamkeit von Opiaten (Uhl GR, et al. The mu opiate receptor as a candidate gene for pain: polymorphisms, variations in expression, nociception, and opiate responses. Proc Natl Acad Sci U S A. 1999 Jul 6;96(14):7752-5).

Die genannten Genveränderungen in spezifischen Rezeptoren können nur in der Weise zur Diagnostik der Wirkungen von Pharmaka benützt werden, als diese Pharmaka spezifische Agonisten oder Antagonisten an den betrachteten Rezeptoren sind. Wünschenswert ist hingegen eine individuelle Diagnostik der generellen Ansprechbarkeit gegenüber allen Pharmaka und die individuelle Vorhersage des Risikos unerwünschter Wirkungen unter Therapie mit Pharmaka.

### Die Diagnostik der Aktivierbarkeit von G-Proteinen erlaubt eine generelle Diagnostik der Wirksamkeit von Pharmaka, deren optimale Dosierung und das Auftreten von Nebenwirkungen.

Unter Pharmaka verstehen wir generell Stoffe, die dem menschlichen Körper von außen zugeführt werden, um bestimmte Zustände herzustelle. Solche Stoffe können Hormone, nieder- oder hochmolekulare.Substanzen, Peptide- oder Proteine, Antikörper u.v.m. sein.

Die meisten zur Behandlung von Krankheiten, körperlichen Fehlfunktionen oder Befindlichkeitsstörungen eingesetzten Pharmaka sind Hormone, Agonisten an Hormonrezeptoren, Antagonisten an Hormonrezeptoren oder andere Substanzen, welche die Expression von Rezeptoren oder die Konzentration von Hormonen direkt oder indirekt beeinflussen. Eine Reihe von Pharmaka übt diesen Einfluss dadurch aus, dass unter Therapie mit solchen Substanzen physiologische Gegenregulationen erfolgen, welche die Konzentrationen von Hormonen erhöhen, die G-Protein-gekoppelte Rezeptoren aktivieren. Als allgemein bekanntes Beispiel sei hier die Therapie mit harntreibenden Substanzen (Diuretika), insbesondere Schleifendiuretika und Thiaziddiuretika genannt. Der bei Therapie auftretende Verlust von Kochsalz und die Blutdrucksenkung führen zur Aktivierung des Renin-Angiotensin-Aldosteronsystems. Das vermehrt gebildete Hormon Angiotensin II stimuliert eine vermehrte Resorption von Natrium in der Niere, stimuliert die Salzaufnahme, erhöht den Blutdruck durch einen direkten vasokonstriktorischen Effekt auf glatte Gefäßmuskelzellen und induziert Proliferationsvorgänge. Es ist allgemein bekannt, dass diese von Angiotensin II hervorgerufenen Mechanismen nach Kopplung des Hormons an Rezeptoren erfolgt, welche Ihre Wirkung über eine Aktivierung heterotrimerer G-Proteine vermitteln. Die Effizienz dieser Wirkungen sind dann vorhersagbar, wenn die Stärke der Aktivierbarkeit von G-Proteinen diagnostiziert werden kann. Andere Pharmaka üben Ihre Wirkung dadurch aus, dass sie die Wiederaufnahme von aus Neuronen freigesetzten Transmittern, z.B. Noradrenalin, Adrenalin, Serotonin oder Dopamin, hemmen. Hier kann als Beispiel das Pharmakon Sibutramin genannt werden, welches im Zentralnervensystem die Wiederaufnahme von Serotonin und Noradrenalin hemmt, dadurch das Hungergefühl reduziert und die Thermogenese steigert. Entsprechend kann Sibutramin zur Therapie der Adipositas eingesetzt werden. Da Noradrenalin und Serotonin G-Protein-gekoppelte Rezeptoren aktivieren, ist die Diagnostik der Aktivierbarkeit von G-Proteinen zur Vorhersage der Wirksamkeit von Sibutramin und dem Auftreten typischer, Sibutramin-assoziierter Nebenwirkungen (z.B. Anstieg von Herzfrequenz und Blutdruck) in besonderem Maße geeignet.

Der Erfindung liegt nun zugrunde, dass ein Verfahren entwickelt wurde, das generell zur Diagnostik der Aktivierbarkeit von G-Proteinen geeignet ist. Hierzu werden eine oder mehrere Polymorphismen im Gen GNAS untersucht, das für die humane Gαs-Untereinheit heterotrimerer G-Proteine kodiert. Besonders geeignet sind hierfür Polymorphismen, welche die Diagnose des Auftretens oder des Nichtauftretens eines alternativen Spleißvorgangs des Gens oder eine geänderte Expression von Gαq vorhersagen. Bei Überexpression kommt es vorhersagbar zu einer gesteigerten Aktivierbarkeit von heterotrimeren G-Proteinen und zur verstärkten Aktivierbarkeit aller Zellen des menschlichen Körpers. Damit erlaubt eine Bestimmung des Vorliegens von Polymorphismen in GNAS die Diagnostik der Wirksamkeit und unerwünschten Wirkungen von Arzneimitteln, insbesondere Agonisten und Antagonisten aller Rezeptoren, deren Wirkungen über heterotrimere G-Proteine vermittelt werden. Daneben können solche Polymorphismen in GNAS dazu verwendet werden, die Wirkungen von Pharmaka zu diagnostizieren, die entweder indirekt oder infolge von Gegenregulationsmechnanismen des Körpers die Konzentrationen von endogenen Hormonen erhöhen oder erniedrigen, deren Rezeptoren heterotrimere G-Proteine aktivieren. Somit erlaubt die Erfindung eine Diagnostik von Wirkungen und unerwünschten Wirkungen aller Pharmaka und beschränkt sich nicht auf Pharmaka die in agonistischer oder antagonistischer Weise spezifische Rezeptoren beeinflussen. Zusätzlich kann die Diagnostik des Allel- oder Haplotypstatus in GNAS dazu eingesetzt werden, die individuell optimale und verträgliche Dosierung von Arzneimitteln zu ermitteln.

Zur Diagnostik einer gesteigerten oder reduzierten Aktivierbarkeit von G-Proteinen dient insbesondere der Nachweise des TT393C-Polymorphismus oder des G(-1211)A-Polymorphismus oder des T(-839)G-Polymorphismus oder des T2291C-Polymorphismus entweder alleine oder in allen denkbaren Kombination.

Daneben können zur Diagnostik alle weiteren Genveränderungen in GNAS verwendet werden, die in einem Kopplungsungleichgewicht zu diesen Polymorphismen stehen und/oder den alternativen Spleißvorgang oder die Expression zusätzlich fördern oder hemmen.

Diese Genveränderungen können mit beliebigen, dem Fachmann geläufigen Verfahren nachgewiesen werden, z.B. direkte Sequenzierung, Restriktionsanalyse, reverse Hybridisierung, Dot-blot- oder slot-blot-Verfahren, Massenspektrometrie, Taqman- oder Light-Cycler-Technologie, Pyrosequencing etc. Ferner können diese Genpolymorphismen gleichzeitig nach Mulitplex-PCR und Hybridisierung an ein DNA-Chip detektiert werden. Daneben können zur Diagnostik einer gesteigerten Aktivierbarkeit von G-Proteinen auch andere Verfahren eingesetzt werden, die den direkten Nachweis des Expressionsniveaus von Gαs oder Spleißvarianten von Gαs ermöglichen.

Das genannte Verfahren eignet sich insbesondere zur Diagnostik der Wirkung von Agonisten oder Antagonisten an Rezeptoren, deren Wirkungen bekanntermaßen von G-Proteinen vermittelt werden. Hierzu werden die folgenden Beispiele genannt, wobei die Liste der Beispiele verlängert werden könnte:
1. Adrenerge Rezeptoren, inbesondere α- und β-Adrenozeptoren und deren Isoformen und Untergruppen, d.h. α1- und α2-Adrenozeptoren sowie β1-, β2-, β3 und β4-Adrenozeptoren
2. Muskarinrezeptoren und deren Isoformen, z.B. m1-,m2-, m3-, m4 und m5-Muskarinrezeptoren und deren Subtypen. Typische Antagonisten an Muskarinrezeptoren sind beispielsweise Atropin, Scopolamin, Ipratroprium, Pirenzepin und N-Butylscopolamin. Typische Agonisten sind Carbachol, Bethanechol, Pilocarpin etc.
3. Dopaminrezeptoren, z.B. D1-, D2-, D3-, D4-, und D5-Rezeptoren und deren Isoformen und Spleißvarianten
4. Serotoninrezeptoren, z.B. 5-HT1- 5-HT2-, 5-HT3-, 5-HT4-, 5HT-5, 5HT-6 und 5-HT7-Rezeptor und deren Subtypen, Isoformen und Spleißvarianten. Typische Agonisten sind Sumatriptan und Cisaprid, Antagonsiten sind beispielsweise Ondansetron, Methysergid, Buspiron und Urapidil.
5. Endothelinrezeptoren und deren Subtypen, Isoformen und Spleißvarianten
6. Bradykininrezeptoren, z.B. B1- und B2-Rezeptoren und deren Subtypen, Isoformen und Spleißvarianten
7. Angiotensinrezeptoren, z.B. AT II Typ1 und Typ2 Rezeptor; typische Antagonisten am AT II-Rezeptor sind Losartan und andere Sartane.
8. Rezeptoren für Endorphine und Opiate, z.B. der µ-Opiatrezeptor
9. Chemokinrezeptoren CCR1-12 und CXCR1-8 für z.B. Interleukin-1/2/3/4/5/6/7/8/9/10/11/12, RANTES, MIP-1α, MIP-1β, stromal cell-derived factor, MCP1-5, TARC, Lymphotactin, Fractalkine, Eotaxin 1-2, NAP-2, LIX etc.
10. Adenosinrezeptoren und deren Subtypen, Isoformen und Spleißvarianten
11. Rezeptoren für Thrombin (Protease-aktivierte Rezeptoren)
12. Rezeptoren für Lyso-Phophatidsäure, Phosphatidsäure, Rezeptoren für Sphingosinphosphate und deren Derivate
13. Rezeptoren für Prostaglandine und Thromboxane, z. B. für PGE1, PGE2, PGF, PGD2, PGI2, PGF2α, Thromboxan A2, etc.
14. Rezeptoren für Neuropetide, z.B. NPY1-5
15. Histaminrezeptoren, z.B. H1-H3-Rezeptoren
16. Rezeptoren für Plättchen-aktivierender Faktor (PAF-Rezeptor)
17. Rezeptoren für Leukotriene
18. Rezeptoren für Insulin, Glucagon, Insulin-like growth factor (IGF-1 und IGF-2), Epidermal Growth Factor (EGF) und platelet-derived growth factor (PDGF)
19. Rezeptoren für Wachstumshormon (GH), Somatostatin (SSTR1-5), thyreotropes Hormon (TSH), Oxytocin, Prolactin, Gonadotropine
20. Rezeptoren für Zytokine, z.B. Interferone
21. Rezeptoren für Purine
22. Orphanrezeptoren, deren Wirkungen durch G- Proteine vermittelt werden.
23. Rezeptoren für Leptin
24. CpG - Oligonucleotide

Vorhergesagt werden können ferner die Wirkungen von Pharmaka, welche die Wiederaufnahme, den Abbau oder die Neusynthese von Neurotransmittern beeinflussen oder bei denen unter Therapie Veränderungen bei der Expression oder Ansprechbarkeit der oben genannten Rezeptoren auftreten (z.B. Sibutramin, Fluoxetin). Diagnostiziert werden können außerdem die Wirkungen aller Pharmaka, welche auf direktem oder indirektem Wege auch in Folge einer physiologischen Gegenreaktion die Konzentrationen von Agonisten, welche die oben genannten Rezeptoren aktivieren, verändern. Vorausgesagt werden kann ferner der Einfluss der Strahlentherapie bei Krebspatienten.

Insbesondere die Wirkungen und unerwünschten Wirkungen der folgenden Pharmaka aus den folgenden Indikationsbereichen können diagnostiziert werden:
1. Antihypertensiva, z.B. β-Blocker (Propanolol, Bisprolol, etc.), Diuretika (Hydrochlorothiazid und weitere Thiaziddiuretika; Furosemid, Piretanid und weitere Schleifendiuretika, Chlorthalidon), α1-Adrenozeptorblocker (z.B. Doxazosin, Prazosin), Angiotensinrezeptor-Blocker (z.B. Losartan), ACE-Hemmer (Enalapril, Captopril, Ramipril etc.), Ca²⁺-Kanalblocker (z.B. Nifedipin, Verapamil, Amlodipin, Felodipin), Clonidin, Reserpin, Renin- Inhibitoren
2. Pharmaka zur Behandlung der Herzinsuffizienz, z.B. β-Blocker (z.B. Propanolol, Metoprolol), ACE-Hemmer (z.B. Captopril, Enalapril, Ramipril, etc.), Angiotensin-Rezeptorblocker (z.B. Losartan), Digitalisglykoside, Katecholamine, Diuretika.
3. Pharmaka zur Behandlung des niedrigen Blutdrucks oder einer Herzinsuffizienz, z.B. α- und β-Smpathomimetika (Effortil, Adrenalin, Noradrenalin, Dobutamin, β-Adrenozeptorblocker, ACE-Hemmer, Angiotensin II-Rezeptorblocker.)
4. Pharmaka zur Behandlung der Migräne, z.B. Sumatriptan, Rizatriptan, Zolmitriptan und weitere Agonisten an Serotoninrezeptoren, β-Blocker (Propanolol, Timolol), Ergotamin und Dihydroergotamin
5. Analgetika vom Morphintyp (Morphin, Codein, etc.)
6. Pharmaka zur Behandlung der koronaren Herzkrankheit wie Adenosin, β-Blocker (z.B. Propanolol, Acebutolol), Nitrate und Ca²⁺-Kanalblocker
7. Pharmaka zur Behandlung psychiatrischer Erkrankungen (Schizophrenie, manisch-depressive Erkrankungen, Psychosen, Depressionen) und von Suchtkrankheiten wie Alkoholismus, (z.B. Fluoxetin, Paoxetin, Imipramin, Desipramin, Doxepin, Mianserin, Trazodon, Lofepramin), Angstsyndrome (Diazepam, etc.), welche z.B. das dopaminerge, serotonerge oder adrenerge System beeinflussen. Aber auch Pharmaka, die ihre Wirkung über Rezeptoren für GABA-, Glycin- oder Glutamat bzw. deren Derivate vermitteln.
8. Pharmaka zur Behandlung des Morbus Alzheimer (z.B. Tacrin) und zur Behandlung des Morbus Parkinson (z.B. Bromocriptin, L-DOPA, Carbidopa, Biperiden, Seleginil, etc.) welche Transmitterkonzentrationen an z.B. muskarinergen oder dopaminergen Substanzen, beeinflussen.
9. Pharmaka zur Behandlung von Asthma bronchiale, welche z.B. entweder direkt bronchodilatierend oder antiinflammatorisch wirken, z.B. Salbutamol, Terbutalin, Albuterol, Theophyllin, Montelukast, Zafirlukast, Cromoglicinsäure, Ipratropiumbromid. Zu solchen Pharmaka gehören auch Antikörper die gegen bestimmte Proteine und Rezeptoren gerichtet sind.
10. Pharmaka zur Behandlung von Motilitätsstörungen des Magens oder Darms und Pharmaka zur Behandlung des Reizdarmsyndroms (irritable bowel syndrome) z.B. N-Butylscopolamin, Pirenzepin, Metoclopramid)
11. Pharmaka zur Behandlung der Adipositas, welche entweder direkt lipolytisch wirksame Rezeptoren aktivieren, z.B. β3-adrenerge Agonisten, oder zentral wirksam sind, z.B. Sibutramin, oder ähnliche Substanzen, die das Sättigungsgefühl verändern oder' welche die Thermogenese beeinflussen. Dazu gehören auch Pharmaka, welche die Magenentleerung beeinflussen.
12. Pharmaka zur Behandlung chronischer Entzündungsvorgänge oder von Störungen des Immunsystems, z.B. Zytokine (Interferone) bei der Therapie von Virushepatitiden oder Interleukin-2 bei HIV-Infektion. Zu solchen Erkrankungen zählen auch Morbus Crohn, Colitis ulcerosa, Asthma, Psoriasis, Neurodermitits, Heuschnupfen. Dazu gehören auch Antikörper gegen Zytokine oder gegen Zytokinrezepotren, z:b. gegen TNFα
13. Pharmaka zur Behandlung der Gestose und der Präeklampsie/Eklampsie und des HELLP-Syndroms
14. Pharmaka zur Behandlung von Fertilitätsstörungen oder zur Beseitigung von Zyklusstörungen bei der Frau oder zur Empfängnisverhütung
15. Pharmaka zur Behandlung von Herzrhythmusstörungen
16. Antidiabetika (Acarbose, Insulin, Troglitazone, Metformin, etc.)
17. Hypnotika, Antiemetika und Antiepileptika
18. Pharmaka zur Behandlung von Störungen des Sexuallebens, z.B. der erektilen Dysfunktion, der weiblichen sexuellen Dysfunktion, Libidomangel, Orgasmusstörungen (Phosphodiesteraseinhibitoren wie Sildenafil, Prostaglandin E1, Agonisten an DopaminRezeptoren, z.B. Apomorphin, Yohimbin, Phentolamin)
19. Pharmaka zur Therapie von Krebserkrankungen und Chemotherapeutika, z.B. 5-Fluoruracil, Antikörper gegen Proteine und Rezeptoren (z.B. gegen HER-2), Substanzen, die Tyrosinkinasen-blockieren, etc.
20. Pharmaka zur Behandlung von allergischen und Tumorerkrankungen, bei denen die Wirkung über die Verabreichung von CpG-Nukleotiden erzielt wird.
21. Pharmaka zur Behandlung des Adipositas, des metabolischen Syndroms oder des Diabetes, z. B.Sibutramin, Orlistat, Leptin, Topiramat, Glinide, Glitazone, Biguanide etc.
22. Pharmaka zur Behandlung der HIV-Infektion, auch Antikörper und Rezeptorblocker. Vorhersage des Entstehens einer Lipodystrophie unter Therapie mit Proteinaseinhibitoren.

Selbstverständlich ist es nicht möglich, im Rahmen der hier beschriebenen Erfindung den Nachweis zu führen, dass alle Pharmakawirkungen durch den GNAS-Genstatus determiniert werden. Naturgemäß kann man auch nicht die genotypabhängigen Wirkungen von Pharmaka untersuchen, die erst in Zukunft entwickelt und eingesetzt werden. Dagegen sollen hier Beispiele für Pharmaka mit unterschiedlichen Wirkmechanismen exemplarisch gezeigt werden, so dass diese Befunde generalisiert werden können.

Beim ersten Pharmakon (Sibutramin) wird der Effekt in der Weise ausgelöst, dass die Wiederaufnahme von Serotonin und Noradrenalin im zentralen Nervensystem blockiert wird. Dadurch wird die Konzentration dieser Transmitter im synaptischen Spalt und extrazellulär erhöht. Diese Transmitter stimulieren nachfolgend G-Protein-gekoppelte Rezeptoren. Die Substanz Sibutramin selbst stimuliert nicht solche Rezeptoren.

Im zweiten Beispiel kommt die Substanz Metformin zum Einsatz. Hierbei handelt es sich um.ein lange bekanntes orales Antidiabetikum mit nicht genau definiertem molekularen Wirkmechanismus. Über eine direkte Beteiligung von G-Proteinen ist nichts bekannt.

Im dritten Beispiel zeigen wir die Wirkung von Isoprenalin, dass bekannterweise direkt G-Protein-gekoppelte Rezeptoren aktiviert.

Wir belegen unsere These einer allgemeinen Vorhersagbarkeit von Pharmakwirkungen damit durch Substanzen, die direkt G-Proteine aktivieren (Isoprenalin), die die Konzentration von Substanzen erhöhen, die nachfolgend G-Proteine erhöhen, und solcher, die keinen Einfluss auf G-Proteine haben.

### Beispiel 1: Wirksamkeit von Sibutramin

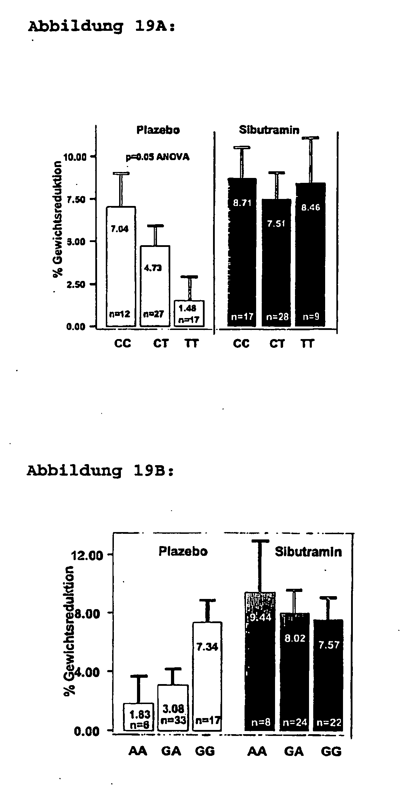

Hier wurden adipöse Patienten im Rahmen einer Plazebokontrollierten, doppelt-blind durchgeführten Studie über 1 Jahr mit 15 mg Sibutramin/die behandelt. Sibutramin ist ein zentral wirksamer Reuptake-Inhibitor von Noradrenalin und Serotonin, die beide ihre Wirkung über G-Protein-gekoppelte Rezeptoren entfalten. Sibutramin (Handelsnamen: Reductil, Meridia) verstärkt das Sättigungsgefühl und erleichtert im Rahmen strukturierter Gewichtsreduktionsmaßnahmen (Steigerung der körperlichen Aktivität, Reduktion der Kalorienaufnahme) das Abnehmen. Klinisches Endziel der Studie war die Gewichtsabnahme nach 1 Jahr. Das genotypabhängige Vergleich Placebo versus Sibutramin ist in Abbildung 19A dargestellt. Abbildung 19A zeigt die Abhängigkeit der Wirkung von Sibutramin gegenüber Placebo im Rahmen der Therapie von Adipositas. Das Placebo bzw. Sibutramin (15 mg/Tag) wurden über 1 Jahr verabreicht. Dargestellt ist die Gewichtsabnahme nach 1 Jahr (%) in Abhängigkeit vom GNAS T393C-Genotyp.
In der Placebogruppe konnten Patienten mit GNAS 393 CC- bzw. CT-Genotyp deutlich besser abnehmen (7,0 und 4,7 %) als diejenigen mit TT-Genotyp (1,5 %). Die Gabe von Sibutramin führt nur bei Trägern des TC- und TT-Genotyps zu einer deutlichen (p = 0,027) Steigerung der Gewichtsabnahme, während dieser günstige Arzneimitteleffekt bei Patienten mit CC-Genotyp nicht beobachtet wurde. Damit ist eine Therapie mit Sibutramin vorwiegend bei Patienten mit TT/TC-Genotyp angezeigt, während Patienten mit CC-Genotyp auch unter Placebo gut abnehmen können.

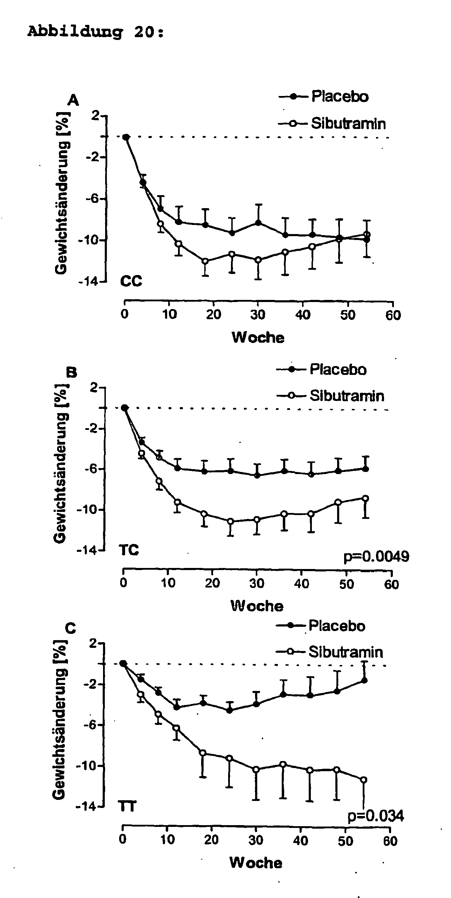

Daneben wurde die Studienpopulation bezüglich des GNAS G(-1211)A Polymorphismus genotypisiert (Abbbildung 19B). Abbildung 19B zeigt die Abhängigkeit der Wirkung von Sibutramin-gegenüber Placebo im Rahmen der Therapie von Adipositas. Placebo bzw. Sibutramin (15 mg/Tag) wurden über 1 Jahr verabreicht. Dargestellt ist die Gewichtsabnahme nach 1 Jahr (%) in Abhängigkeit vom GNAS G(1211)A-Genotyp. In der Placebogruppe konnten Patienten mit GNAS (-1211) GG-Genotyp deutlich besser abnehmen (7,3 %) als diejenigen mit AA- und AG-Genotyp (1,8 und 3,1 %) Die Gabe von Sibutramin führt nur bei Trägern des AG- und AA-Genotyps zu einer deutlichen (p = 0,027) Steigerung der Gewichtsabnahme, während dieser günstige Arzneimitteleffekt bei Patienten mit GG-Genotyp nicht beobachtet wurde. Damit ist eine Therapie mit Sibutramin vorwiegend bei Patienten mit AG/AA-Genotyp angezeigt, während Patienten mit CC-Genotyp auch unter Placebo gut abnehmen können. Die Zeitverläufe der Gewichtsabnahme für jeden Genotyp des 393CT393C-Polymorphismus (Sibutramin versus Placebo) sind in den Abbildungen 20A, 20B und 20C dargestellt. Die Abbildungen 20 A, B und C zeigen im einzelnen die Zeitverläufe der Gewichtsveränderungen unter Placebo bzw. Sibutramin für den GNAS 393 CC Genotyp (A), den TC-Genotyp (B) und den TT-Genotyp (C). Man sieht, dass nur TC- und TT-Genotypen von einer Therapie mit 15 mg Sibutramin täglich profitieren, während CC-Genotypen ohne Medikament abnehmen können.

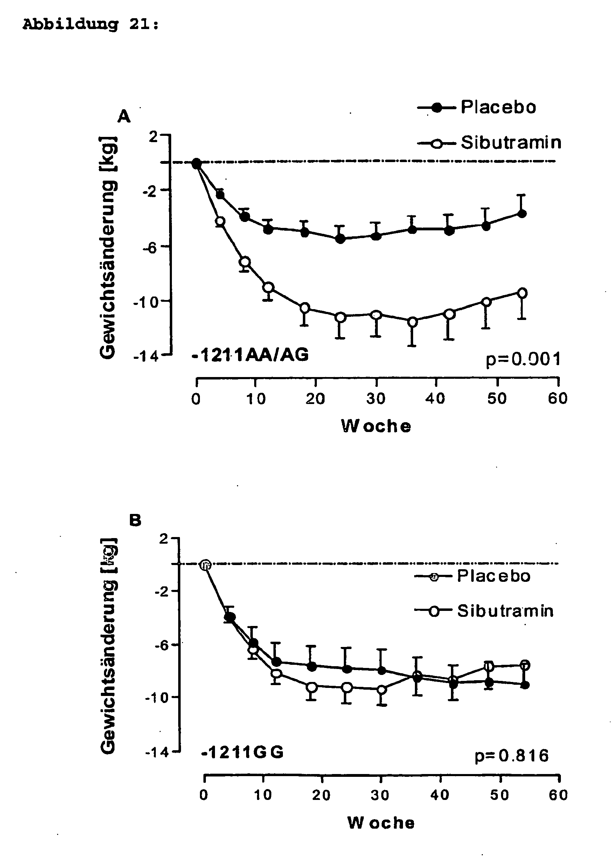

Die Zeitverläufe der Gewichtsabnahme für jeden Genotyp des G(-1211)A-Polymorphismus (Sibutramin versus Placebo) sind in den Abbildungen 21A und 21B dargestellt. Die Abbildungen 21A und B zeigen im einzelnen die Zeitverläufe der Gewichtsveränderungen unter Placebo bzw. Sibutramin für den GNAS -1211 AA/AG Genotyp (A) und den GG-Genotyp (B). Man sieht, dass nur AA/AG-Genotypen von einer Therapie mit 15 mg Sibutramin täglich profitieren, während GG-Genotypen ohne Medikament abnehmen können.

Damit wird gleichzeitig gezeigt, dass auch die Vorhersage des Erfolgs nicht-pharmakologischer Maßnahmen zur Gewichtsreduktion unter Verwendung von Genveränderungen im Gen GNAS möglich sind. Dazu gehören strukturierte Gewichtsabnahmeprogramme (z.B. optifast, Weight Watchers, andere Schulungsprogramme), die Einnahme von sättigenden Quellstoffen (CM3, BMI23) oder von kalorienreduzierten Nahrungsmitteln.

### Beispiel 2: Wirksamkeit oraler Anti-Diabetika zur Gewichtsabnahme und zur Verbesserung der Insulinsensitivität

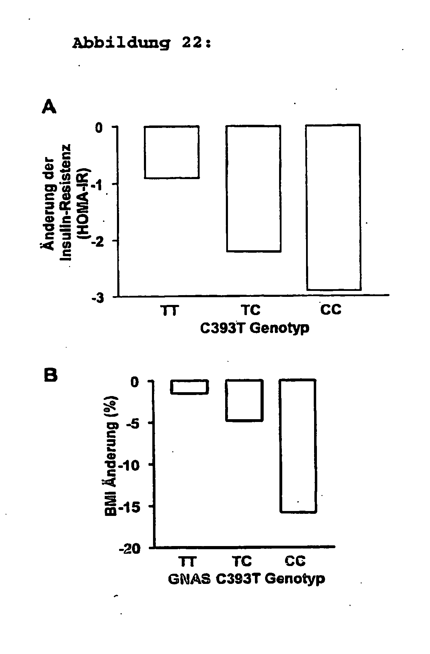

Das Polycystische Ovarial Syndrom (PCOS) ist eine häufige endokrine Erkrankung, die durch chronische Anovulation und Hyperandrogenismus charakterisiert ist. Etwa 5 % aller prämenopausalen Frauen sind davon betroffen. Die meisten Frauen mit PCOS haben eine Insulinresistenz und damit ein erhöhtes Risiko für ein metabolisches Syndrom (Adipositas, Typ- 2 Diabetes, Fettstoffwechselstörung und Hypertonie). Die Behandlung von PCOS- Patienten mit Metformin verbessert die Fertilität, Androgen level, reduziert die Insulinresistenz und erleichtert eine Gewichtsreduktion. Hier wurden Frauen mit PCOS-Syndrom über 12 Monate mit Metformin behandelt. Dabei wurde die Änderung des Body Mass Index (BMI) und der Insulinresistenz nach der HOMA-IR-Methode (homeostasis model assessment for insulin resistance)mit GNAS-Genotypen des T393C-Polymorphismus untersucht. Abbildung 22A zeigt den Einfluß einer Therapie mit einem oralen Anti-Diabetikum (Metformin ) auf Insulinresistenz (A) und Body Mass Index (BMI; B) bei Frauen mit polycystischen Ovarien. Die Therapie erfolgte, wie bereits erwähnt, mit Metformin über 12 Monate. Dargestellt ist die Änderung des HOMA-IR Index als Maß für die Insulinresistenz. Deutlich zu erkennen ist die stärkste Verbesserung des HOMA-IR-Index bei Individuen mit CC-Genotyp (2,9) im Gegensatz zu 2,2 bei TC- und nur 0,9 bei TT-Genotypen. Damit zeigen CC und TC- Genotypen unter Metformin-Therapie die deutlichste Zunahme der Insulinsensitivität, während TT-Genotypen davon nur gering profitieren. In gleicher Weise verhalten sich die Änderungen des BMI (Maß für das Körperfett). Hier erfolgt eine Abnahme um 15,8 % beim CC-Genotyp, während der BMI bei TC- und TT-Genotypen um nur 4,8 bzw. 1,5 % reduziert wurde (Abbildung 22B). Damit ist gezeigt, dass Genveränderungen im Gen GNAS dazu verwendet werden können, die Wirksamkeit oraler Antidiabetika vorherzusagen,. Dazu gehören neben Metformin die sog. Glitazone, Tolbutamid, Sulfonylharnstoffe und seine Derivate, Glinide sowie Arcabose.

### Beispiel 3: Vorhersage der Wirkung von Katecholaminen

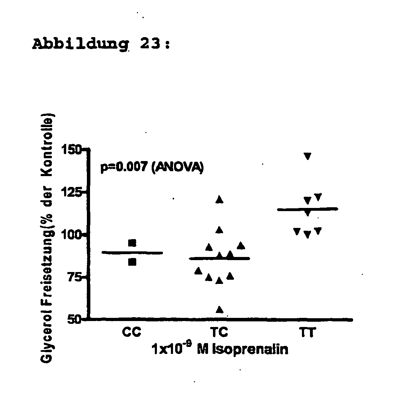

Hierzu wurden menschliche Fettzellen ex vivo mit Isoprenalin stimuliert und die Lipolyse anhand der Freisetzung von Glycerol quantifiziert (Hauner H et al. Effects of the G-protein beta3 subunit 825T allele on adipogenesis and lipolysis in cultured human preadipocytes and adipocytes. Horm Metab Res. 2002;34(9):475-80). Hier zeigt sich die stärkste Stimulation der Lipolyse bei Fettzellen von Individuen mit dem GNAS 393 TT -Genotyp (Abbildung 23). Dies stimmt mit der Beobachtung einer vermehrten Expression von Gαs-Protein bei diesem Genotyp überein.

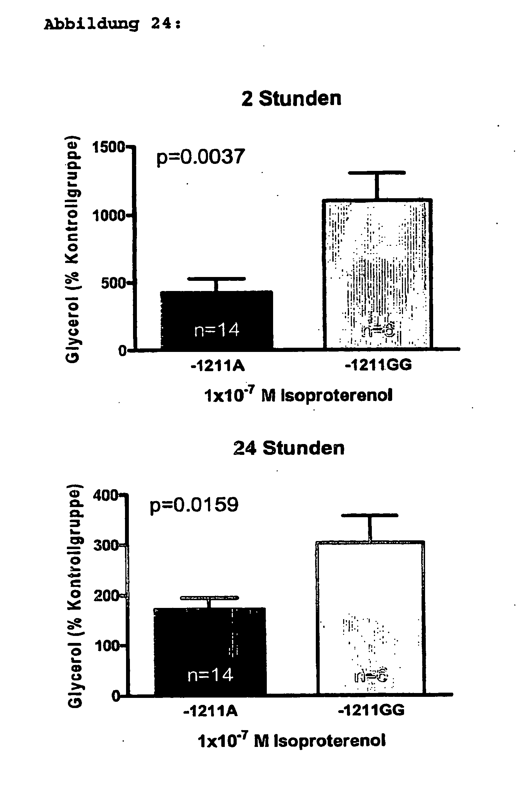

Für den G(-1211)A Polymorphismus zeigte sich die stärkste Stimulation der Lipolyse bei GG Genotypen (Abbildung 24). Abbildung 24 zeigt die Isoprenalin-stimulierte Lipolyse in humanen Fettzellen in Abhängigkeit von GNAS G(-1211)A -Status. In Fettzellen von Menschen mit GG-Genotyp findet man hier eine gesteigerte Lipolyse, gemessen als Freisetzung von Glycerol. Da für diesen Polymorphismus keine Korrelation zu veränderter Expression zu erkennen war, wird hier ein anderer Wirkmechanismus als bei dem T393C Polymorphismus vermutet, da diese außerdem mit der Beobachtung übereinstimmt, dass Personen mit GG Genotyp unter kalorienreduzierter Diät mehr Gewicht verlieren als Personen mit AA/AG Genotyp.

Hiermit wird gezeigt, dass Genveränderungen im GNAS-Gen dazu verwendet werden können, die Wirkung von Katecholaminen vorherzusagen. Dies betrifft demnach Substanzen, die direkt α- und/oder β- Adrenozeptoren stimulieren und auch Substanzen, welche eine vermehrte Freisetzung von Katecholaminen induzieren. Daneben können Nebenwirkungen vorhergesagt werden, wobei durch Gabe beliebiger Substanzen eine Sympathikusaktivierung verursacht wird (Sildenafil und ähnliche PDE-Hemmer).

### Beispiel 4: Vorhersage von Kardiovaskulären Nebenwirkungen unter Therapie mit Sibutramin

Aufgrund des Wirkmechanismus (zentrale Hemmung der Wiederaufnahme von Noradrenalin und Serotonin) kommt es unter der Einnahme von Sibutramin zu typischen Nebenwirkungen wie Mundtrockenheit, Schlafstörungen und Obstipation. Gefährlicher sind jedoch die kardiovaskulären Nebenwirkungen, z.B. Anstieg der Herzfrequenz und des Blutdrucks, die zu Tachykardien und zum Myokardinfarkt führen können. Bislang ist keine Vorhersage dazu möglich, bei welchen Personen dies auftritt.

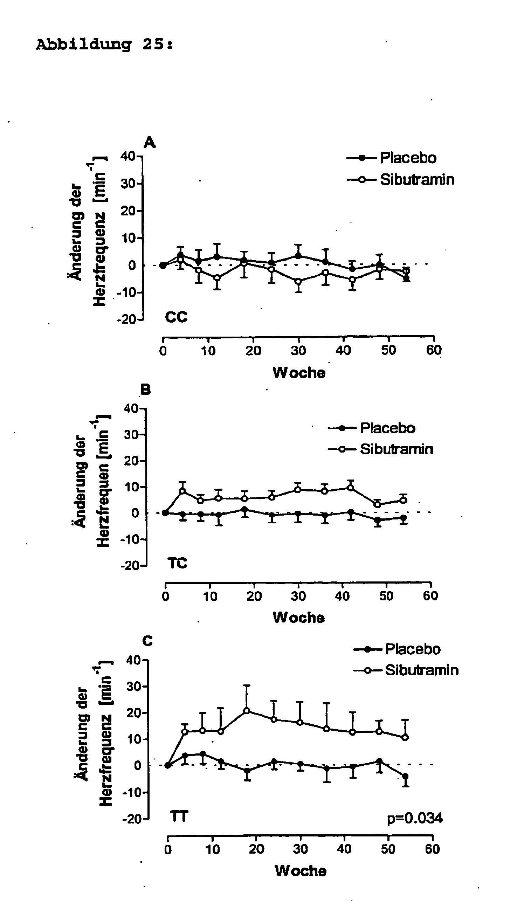

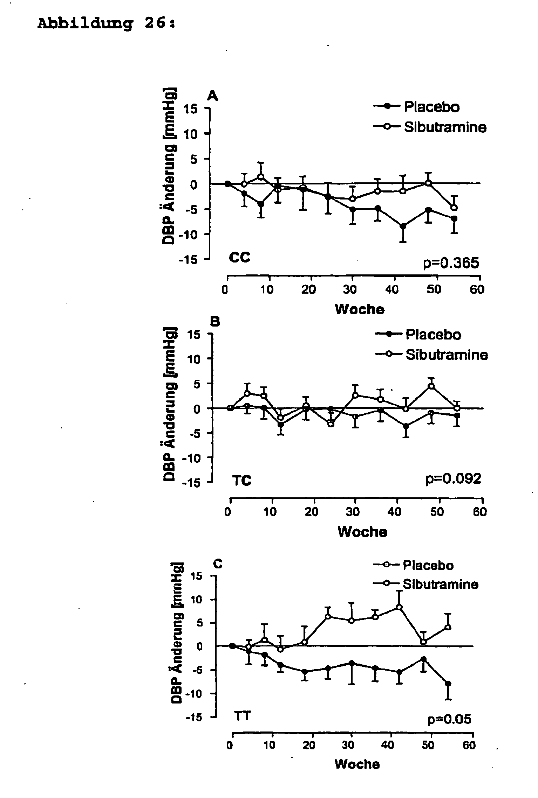

Es wurde hier der Zusammenhang von Genveränderungen im Gen GNAS mit der Änderung von Herzfrequenz unter Einnahme von Sibutramin versus Placebo untersucht (Abbildung 25). Dargestellt sind hier die Effekte in Abhängigkeit von Genotypen des T393C-Polymorphismus. Man erkennt bei T393C- (Abbildung 25B) und TT-Genotypen (Abbildung 25C) eine durch Sibutramin (versus Placebo) signifikant gesteigerte Herzfrequenz um 10 (TC) bis zu 20 Schläge pro Minute. Dieser Effekt tritt bei CC-Genotypen nicht auf (vgl. Abbildung 25A). Abbildung 26 verdeutlicht die mögliche Verwendung einer Genveränderungen im humanen GNAS Gen zur Vorhersage eines Anstiegs des diastolischen Blutdrucks unter Therapie mit Sibutramin. Dargestellt sind die zeitabhängigen Veränderungen des diastolischen Blutdrucks (DBP) über die Zeit in Abhängigkeit von Genotypen des T393C-Polymorphismus. Wie in Abbildung 26 dargestellt beobachtet man ferner unter Therapie mit Sibutramin einen signifikanten Anstieg des Blutdrucks bei 393 TT Genotypen (Abbildung 26C), der bei CC- (Abbildung 26A) und TC-Genotypen (Abbildung 26B) nicht auftritt.

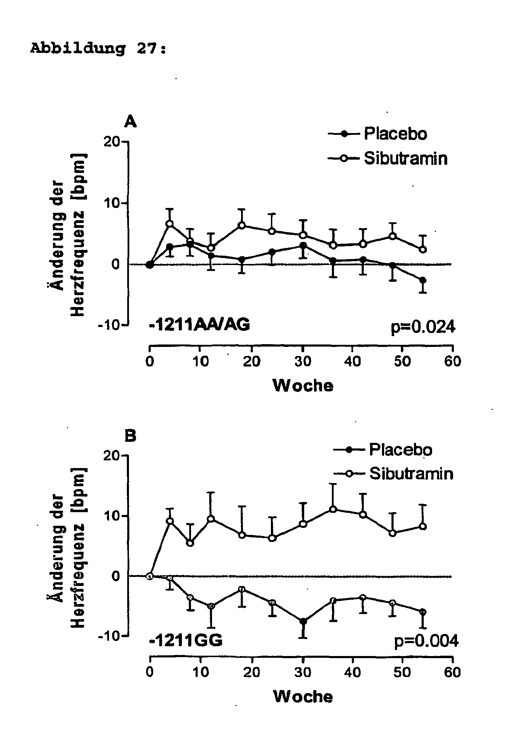

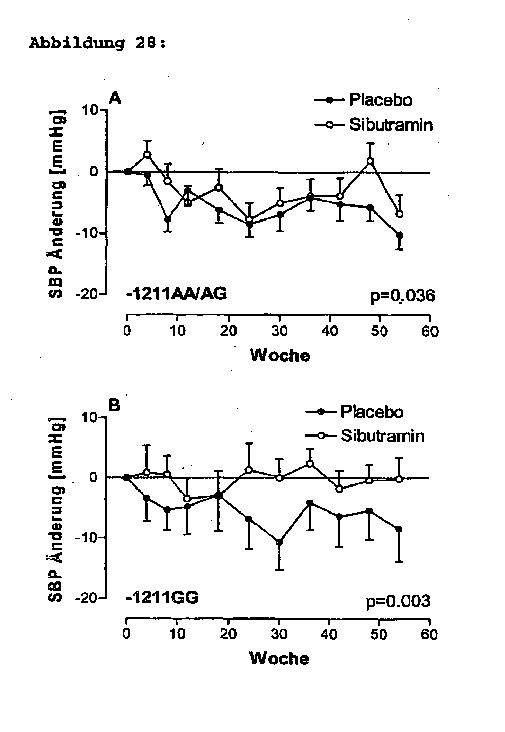

Abbildung 27 verdeutlicht nochmals die mögliche Verwendung einer Verwendung einer Genveränderungen im humanen GNAS Gen zur Vorhersage eines Anstiegs der üerzf requenz unter Therapie mit Sibutramin. Dargestellt sind hier die Effekte in Abhängigkeit von Genotypen des G(-1211)A-Polymorphismus. Für den G(-1211)A Polymorphismus erkennt man bei GG Genotypen (Abbildung 27B) gegenüber AA/AG-Genotypen (Abbildung 27C) eine durch Sibutramin (versus Placebo) signifikant stärke Änderung der Herzfrequenz. Abbildung 28 schließlich verdeutlicht die Möglichkeit einer weiteren Verwendung einer Genveränderungen im humanen GNAS Gen zur Vorhersage eines Anstiegs des systolischen Blutdrucks unter Therapie mit Sibutramin. Dargestellt sind die zeitabhängigen Veränderungen des systolischen Blutdrucks (SBP) über die Zeit in Abhängigkeit von Genotypen des G(-1211)A-Polymorphismus. Hierbei beobachtet man unter Therapie mit Sibutramin ein fehlendes Absinken des systolischen Blutdruckes -1211 GG Genotypen (Abbildung 28B), der Unterschied der Bluztdruckveränderung ist signifikant höher als bei AA/AG Genotypen (Abbildung 28A). Diese Beobachtungen sind therapeutisch deshalb so bedeutsam, da gezeigt wurde, dass gerade Patienten mit GG Genotypen durch kalorienreduzierte Ernährung sowie Lebensstiländerung allein ausreichend Gewicht verlieren, diese also nicht von einer Therapie mit Sibutramin profitieren.

Die Verwendung von Genveränderungen ist daher dazu geeignet, Patienten zu identifizieren, bei denen unter Therapie mit Medikamenten kardiovaskuläre Nebenwirkungen auftreten. Diese Effekte können direkt verursacht werden, indem solche Medikamente Rezeptoren stimulieren, die Gαsvermittelt eine Vasokonstriktion oder eine Herzfrequenzsteigerung hervorrufen. Dazu gehören beispielsweise Sibutramin, Triptane und Noradrenalin-/Serotonin-Wiederaufnahmehemmer. Ferner gehören dazu Medikamente, die den Abbau von Katecholaminen hemmen (MAO-Hemmer) und trizyklsiche Antidepressiva. Ferner gehören dazu Medikamente, die einen Blutdruckabfall bewirken und reflektorisch eine Aktivierung des Sympathikus induzieren (z.B. Sildenafil und andere Inhibitoren von Phosphodiesterasen, Nitrate).

Ein weiterer Beleg für die generelle Anwendung von Genveränderungen im Gen GNAS zur Vorhersage von Pharmakawirkungen ergibt sich auch aus den beobachteten Genotyp abhängigen Krankheitsverläufen bei Harnblasenkarzinom (Abbildung 15), Nierenzellkarzinom (Abbildung 16) und akuter myeloischer Leukämie (Abbildung 17). Diese Patienten wurden alle mit unterschiedlichen Pharmaka behandelt. Die durch Verwendung von Genveränderungen im Gen GNAS sichtbar gemachten unterschiedlichen Krankheitsverläufe belegen ein unterschiedliches Ansprechen auf diese Therapieformen .

### Einordnung der Erfindung

Es werden Polymorphismen im Gen GNAS beschrieben, die untereinander im Kopplungsungleichgewicht stehen und alleine oder in beliebigen Kombinationen zur Vorhersage von Krankheitsverläufen oder Wirkungen von Pharmaka verwendet werden können. Der erste Teil der Erfindung besteht aus dem Nachweis der Genveränderungen und dem Nachweis von Haplotypen bzw. Kopplungsungleichgewichten. Der zweite Teil der Erfindung beschreibt die Verwendung der Genveränderungen zur Vorhersage für Erkrankungsrisiken und Krankheitsverläufen. Grundlage dafür ist die geänderte Aktivierbarkei.t von Gαs auf Grund der beschrieben Genveränderungen. Diese bestimmen dann gleichzeitig ein vom Genstatus abhängiges unterschiedliches Ansprechen auf Pharmaka. Hier stellt sich die Frage, ob die gleichzeitige Verwendung von Genveränderungen für die beschriebenen Zwecke unerwartet ist, oder ob diese dem Fachmann bekannt ist. Dies soll an einem Beispiel demonstriert werden.

Dieser enge Zusammenhang wurde bereits für Genveränderungen im Gen GNB3 beschrieben. Auch mit diesen Genveränderungen, die zur Funktionsveränderung einer Gβ3-Untereinheit führen, können gleichzeitig Krankheitsrisiken, Krankheitsverläufe aber auch das Ansprechen auf unterschiedliche Pharmaka vorhergesagt werden. Ein C825T-Polymorphismus im Gen GNB3 steht im Kopplungsungleichgewicht mit weiteren Polymorphismen im selben Gen, so dass spezifische Haplotypen beschrieben werden können (D. Rosskopf et al., Identification and ethnic distribution of major haplotypes in the gene GNB3 encoding the G-protein beta3 subunit. Pharmacogenetics 12 (3):209-220, 2002). Diese Genveränderungen sind mit einem erhöhten Risiko für ganz unterschiedliche Erkrankungen assoziiert, z.B. Bluthochdruck, Adipositas, Schlaganfall, Herzinfarkt, Insulinresistenz, Diabetes, aber auch Depression (P. Zill et al., Evidence for an association between a G-protein beta3-gene variant with depression and response to antidepressant treatment. Neuroreport 11 (9):1893-1897, 2000.; W. Siffert et al., G protein b3 subunit 825T allele and its potential association with obesity in hypertensive subjects. J.Hypertens. 17:1095-1098, 1999.; W. Siffert et al. Association of a human G-protein beta3 subunit variant with hypertension. Nat. Genet. 18 (1):45-48, 1998; R. A. Hegele et al., G-protein beta3 Subunit Gene Splice Variant and Body Fat Distribution in Nunavut Inuit. Genome Res. 9 (10):972-977, 1999.C. K. Naber, et al., Interaction of the ACE D Allele and the GNB3 825T Allele in Myocardial Infarction. Hypertension 36 (6):986-989, 2000; A. C. Morrison et al., G-Protein beta3 Subunit and alpha-Adducin Polymorphisms and Risk of Subclinical and Clinical Stroke. Stroke 32 (4):822-829, 2001. T. C. Wascher et al. Associations of a human G protein beta3 subunit dimorphism with insulin resistance and carotid atherosclerosis. Strake 34 (3):605-609, 2003. D. Rosskopf et al. Interaction of the G Protein beta3 Subunit T825 Allele and the IRS-1 Arg972 Variant in Type 2 Diabetes. Eur.J.Med.Res. 5 (11):484-490, 2000.)

Gleichzeitig sind diese Genveränderungen im Gen GNB3 mit dem Ansprechen auf ganz unterschiedliche Pharmaka assoziiert. Dazu gehören auch solche, deren Wirkung nicht direkt durch G-Proteine vermittelt werden. Als Beispiele sind zu nennen: Hyhdrochlorothiazid (Wirkung nicht direkt durch G-Protein vermittelt), Sildenafil (Wirkung nicht direkt G-Protein vermittelt), Impfung gegen Hepatitis B (Wirkung nicht direkt durch G-Protein vermittelt), unterschiedliche Antidepressiva (Wirkung nicht direkt durch G-Protein vermittelt), Sibutramin (Wirkung nicht direkt durch G-Protein vermittelt), Antidiabetika (Wirkung nicht direkt durch G-Protein vermittelt), Pharmaka zur Behandlung der Leukämie (Wirkung nicht direkt durch G-Protein vermittelt). Daneben findet man erwartungsgemäß eine Vorhersage der Wirkung von Pharmaka, die G-Protein gekoppelte Rezeptoren aktivieren oder hemmen, z.B. von Clonidin, Propanolol, Noradrenalin, Endothelin, Angiotensin etc. (J. M. Fernandez-Real et al, G Protein beta3 Gene Variant, Vascular Function, and Insulin Sensitivity in Type 2 Diabetes. Hypertension 41 (1):124-129, 2003; P. Zill et al; Evidence for an association between a G-protein beta3-gene variant with depression and response to antidepressant treatment. Neuroreport 11 (9):1893-1897, 2000. R. R. Wenzel et al., Enhanced vasoconstriction to endothelin-1, angiotensin II and noradrenaline in carriers of the GNB3 825T allele in the skin microcirculation. Pharmacogenetics 12 (6):489-495, 2002. S. T. Turner et al., C825T Polymorphism of the G Protein beta(3)-Subunit and Antihypertensive Response to a Thiazide Diuretic. Hypertension 37 (2 Part 2):739-743, 2001. R. F. Schäfers et al., Haemodynamic characterization of young normotensive men carrying the 825T-allele of the G-protein beta3 subunit. Pharmacogenetics 11 (6):461-470, 2001. M. Ryden et al., Effect of the (C825T) Gbeta(3) Polymorphism on Adrenoceptor-Mediated Lipolysis in Human Fat Cells. Diabetes 51 (5):1601-1608, 2002. H. Hauner et al., Prediction of successful weight reduction under sibutramine therapy through genotyping of the G-protein beta3 subunit gene (GNB3) C825T polymorphism. Pbarmacogenetics 13 (8):453-459, 2003. OH. J. Lee et al., Association between a G-protein beta3 subunit gene polymorphism and the symptomatology and treatment responses of major depressive disorders. Pharmacogenomics.J., 2003. A. Mitchell et al., Venous response to nitroglycerin is enhanced in young, healthy carriers of the 825T allele of the G protein beta3 subunit gene (GNB3). Clin.Pharmacol.Ther. 74 (5):499-504, 2003. H. Nuckel et al., The CC genotype of the C825T polymorphism of the G protein beta 3 gene (GNB3) is associated with a high relapse rate in patients with chronic lymphocytic leukaemia. Leukemia & Lymphoma 44 (10):1739-1743, 2003. J. Nürnberger et al., Effect of the C825T polymorphism of the G protein beta3 subunit on the systolic blood pressure-lowering effect of clonidine in young, healthy male subjects. Clin.Pharmacol.Ther. 74 (1):53-60, 2003. A. Serretti et al., SSRIs antidepressant activity is influenced by Gbeta3 variants. Eur.Neuropsychopharmacol. 13 (2):117-122, 2003. H. Sperling et al., Sildenafil Response is Influenced by the G Protein beta3 Subunit Gnb3 C825t Polymorphism: A Pilot Study. J.Urol. 169 (3):1048-1051, 2003. M. Lindemann et al., Role of G protein beta3 subunit C825T and HLA class II polymorphisms in the immune response after HBV vaccination. Virology 297 (2):245-252, 2002)

Es ist demzufolge davon auszugehen, dass die vorliegend beschriebenen Zusammenhänge zwischen den beschriebenen Genveränderungen im humanen Gen GNAS und der Vorhersage von Krankheitsrisiken, Krankheitsverläufen und von Ansprechen auf Krankheitstherapien mittels pharmakologischer und nicht-pharmakologischer Maßnahmen und zur Vorhersage unerwünschter Arzneimittelwirkungen auch insoweit vorhanden sind, als diese vorliegend nicht durch wissenschaftliche Untersuchungen belegt wurden.

## Patentansprüche

1. In vitro Verfahren zur Vorhersage des Krankheitsrisikos, des Krankheitsverlaufs und des Ansprechens und der Nebenwirkungen von Arzneimitteln bei Krankheiten, die mit einer Genveränderung im humanen Gen GNAS assoziiert sind, bei welchem eine Genveränderung im Gen für humanes G-Protein Gαs-Untereinheit identifiziert wird, **dadurch gekennzeichnet, dass** man
nach der Genveränderung T393C im GNAS Gen sucht und die Genveränderung zur Beurteilung des Risikos von Adipositas und/oder Insulin-Resistenz, zur Vorhersage des Krankheitsverlaufs einer Krebserkrankung, des Ansprechens eines Arzneimittels bei Adipositas sowie zur Vorhersage der Nebenwirkungen von Wiederaufnahmeinhibitoren, von Serotonin und Noradrenalin verwendet
oder
nach einer oder mehreren Genveränderung/en in der Promotorregion und/oder im Intron 1 des Gens GNAS, ausgewählt aus G(-1211) A A2025G, C2273T und 1340del, sucht und die Genveränderungen zur Vorhersage des Krankheitsverlaufs einer Krebserkrankung oder einer kardiovaskulären Erkrankung, des Ansprechens eines Arzneimittels bei Adipositas, sowie zur Vorhersage der Nebenwirkungen von Wiederaufnahmeinhibitoren von Serotonin und Noradrenalin verwendet.

2. Verfahren nach Anspruch 1, **dadurch gekennzeichnet, dass** man nach einer Kombination der Genveränderungen sucht.

## Claims

1. In vitro method for the prediction of the risk of disease, the course of disease, and the response and the side-effects of medicaments for diseases that are associated with a gene alteration in the human gene GNAS, whereby an alteration in the gene for human G-protein Gαs-subunit is identified, **characterized in that** one searches for the gene alteration T393C in the GNAS gene and uses this gene alteration for the evaluation of the risk of obesity and/or insulin resistance, for the prediction of the course of disease of a cancer, the response of a medicament for obesity and for the prediction of the side-effects of reuptake inhibitors of serotonin and noradrenalin
or
one searches for one or more gene alteration/s in the promoter region and/or in Intron 1 of the gene GNAS, selected from G(-1211)A, A2025G, C2273T and 1340del, and uses the gene alteration/s for the prediction of the course of disease of a cancer or a cardiovascular disease, the response of a medicament in case of obesity and for the prediction of the side-effects of reuptake inhibitors of serotonin and noradrenalin.

2. The method according to claim 1, **characterized in that** one searches for a combination of the gene alterations.

## Revendications

1. Procédé in vitro pour la prévision du risque pathologique, de l'évolution de la pathologie et de la réaction aux effets secondaires de médicaments, lors de maladies associées à une mutation génétique du gène humain GNAS, procédé dans lequel une mutation génétique est identifiée dans le gène pour la sous-unité Gαs de la protéine G, **caractérisé en ce que**
l'on recherche la mutation génétique T393C dans le gène GNAS et utilise la mutation génétique pour la prévision du risque d'adiposité et / ou de résistance à l'insuline, pour la prévision du déroulement d'une maladie cancéreuse, la réponse à un médicament en cas d'adiposité, ainsi que la prévision des effets secondaires d'inhibiteurs de recaptage de la sérotonine et de la noradrénaline
ou
l'on recherche une ou plusieurs mutation/s génétique/s dans la région promotrice et / ou dans l'intron 1 du gène GNAS, choisis parmi G(-1211)A, A2025G, C2273T et 1340del, et utilise la/les mutation/s génétique/s pour la prévision du déroulement d'une maladie cancéreuse ou d'une maladie cardiovasculaire, la réponse à un médicament en cas d'adiposité, ainsi que la prévision des effets secondaires d'inhibiteurs de recaptage de la sérotonine et de la noradrénaline.

2. Procédé selon la revendication 1, **caractérisé en ce que** l'on recherche une combinaison des mutations génétiques.
